(19) Europäisches Patentamt
European Patent Office
Office européen des brevets

(11) Veröffentlichungsnummer: **0 083 600**
**B1**

(12) # EUROPÄISCHE PATENTSCHRIFT

(45) Veröffentlichungstag der Patentschrift: **25.11.87**

(21) Anmeldenummer: **82901956.1**

(22) Anmeldetag: **08.07.82**

(86) Internationale Anmeldenummer: **PCT/AT 82/00021**

(87) Internationale Veröffentlichungsnummer: **WO 83/00486 (17.02.83 Gazette 83/05)**

(51) Int. Cl.⁴: **C 07 F 9/65**, C 07 F 9/56, C 07 F 9/22, A 61 K 31/66, A 61 K 49/00 // A61K43/00

(54) **Verwendung von Alkaloidderivaten zur Herstellung eines Arzneimittels zur Stärkung des Immunsystems und zur Behandlung von viralen,bakteriellen und Pilzinfektionen.**

(30) Priorität: **13.07.81 DE 3128018**

(43) Veröffentlichungstag der Anmeldung: **20.07.83 Patentblatt 83/29**

(45) Bekanntmachung des Hinweises auf die Patenterteilung: **25.11.87 Patentblatt 87/48**

(84) Benannte Vertragsstaaten: **AT BE CH DE FR GB LI LU NL SE**

(56) Entgegenhaltungen:
**AT-B-354 644**
**FR-A-2 366 020**
**US-A-3 865 830**

**Der Grosse Brockhaus F.A.Brockhaus, Wiesbaden, 18. Auflage, Bd. 4, S. 405 und Bd. 8, S. 42**

**Die Akte enthält technische Angaben, die nach dem Eingang der Anmeldung eingereicht wurden und die nicht in dieser Patentschrift enthalten sind.**

(73) Patentinhaber: **Nowicky, Wassyl, Laimgrubengasse 19/5, A-1060 Wien (AT)**

(72) Erfinder: **Nowicky, Wassyl, Laimgrubengasse 19/5, A-1060 Wien (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 083 600 B1

LIBER, STOCKHOLM 1987

**Beschreibung**

Die Erfindung betrifft die Verwendung von Alkaloidderivaten zur Herstellung eines Arzneimittels zur Stärkung des Immunsystems und zur Behandlung von viralen, bakteriellen und Pilz-Infektionen.

Aus US-A-3865830, FR-A-2366020 und AT-A-354644 ist ein Verfahren zur Herstellung von Salzen von Alkaloidderivaten der Thiophosphorsäure bekannt, deren pharmakologische Wirksamkeit als Cytostatika und Mutagen erkannt worden ist und für die wasserlösliche Verbindungen in vorteilhafter Weise vorgeschlagen worden sind. Als wasserlösliche Alkaloidsalze kommen Berberin, Sanguinarin sowie Salze der Alkaloide des großen Schöll-Krautes in Frage und Salze der Alkaloide Bisbenzylisochinolin-Alkaloide, z. B. Curin, Fangohinolin, Tetrandin, Pendulin, Thalidasine, Aporfin-Benzylischinolin-Alkaloide z. B. Thalicarpin, Ibogo-Alkaloide, z. B. 20-Hydroxyvoacamidin, Indol-Indolin-Alkaloide z. B. Leorosidin, Lurosin, Vinkaleukoblastin, Leurocristin, Tropolon-Alkaloide z. B. Colchicin, Isochinolin-Alkaloide z. B. Liriodenin, 0-Methylatherolin, Oxopurpurin, Chelidonin, Protpin, Stylopin, Allokryptopin, Coptisin, Chelerytrin, Corysamin, Chelidimerin, Homochelidonin, Methoxychelidonin, Chelilutin, Chelirubin, Narciclasin, Talicarpin, Pakistanien, Pacistanamine, Pensylwanin, Pwesylwanamin, Berberin, Sanguinarin, Coffein, Nitydyn, Faraganin, Steroid-Alkaloide, Indol-Isochinolin-Alkaloide z. B. 9-Methoxy-ellipticin, Ellipticin, Indol-Alkaloide z. B. Reserpin, Chinolin-Indolizidin-Alkaloide z. B. Compothecin, Pyrolin-Alkaloide z. B. Tatrofan, Pyrolizidin-Alkaloide z. B. Heliotrin, Acridon-Alkaloide z. B. Melicopin, Acromycin, Normelioepidin, Phenanthroindolizidin-Alkaloide z. B. Tylopherin, Tylocrebin, Imidazol-Alkaloide z. B. Pilocarpin, Chinolizidin-Alkaloide z. B. Matrin, Oxymarin, Cryptoleurin, Chinazolon-Alkaloide z. B. Febrifugin, Benzuzepin-Alkaloide z. B. Cephalotaxin, Deoxyharringtonin, Homoharringtonin, Harringtonin und andere.

Auch die Thiophosphorderivate von Alkaloiden in Form freier Basen sind in dem hier interessierenden Zusammenhang bekannt. Beispiele derartiger bekannter Derivate sind Thiophosphorsäuredi(äthylenimido)-N-berberinol-äthylamid, Thiophosphorsäuretri(N-sanguinarinol)-äthylamid sowie Thiophosphorsäureamidoderivate der Gesamtalkaloide der kondensierten Isochinolinsysteme des Großen Schöllkrautes.

Diese Verbindungen mit ihrer bekannten cytostatischen Wirksamkeit sind für sich in Wasser nur schwer löslich, wobei die Verwendung von Wasser als Lösungsmittel insbesondere für die Zubereitung von Injektionslösungen stets der Verwendung von organischen Lösungsmitteln für den gleichen Zweck vorzuziehen ist.

Die gewünschte Wasserlöslichkeit erfolgt, ohne daß die Wirkstoffe dabei ihre cytostatische Wirkung einbüßen bzw. unerwünschte Nebenwirkungen auftreten, dadurch, daß das jeweilige Alkaloid mit einem anderen aus der Gruppe der Alkylantien, Antimetaboliten und Antibiotika sowie anderer stickstoff- oder phosphorhaltiger organischer Verbindungen umgesetzt und das erhaltene Produkt gegebenenfalls in ein pharmazeutisch verwendbares Salz überführt wird. Als Alkaloidkomponente haben sich die vorgenannten Verbindungen als besonders geeignet erwiesen.

Als Mittel für die Umsetzung kommen insbesondere in Frage:

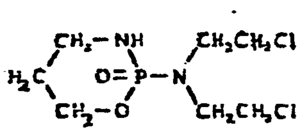

(XXXIX)

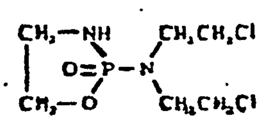

(XL)

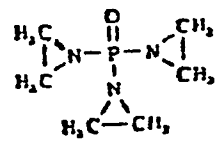

(LIV)

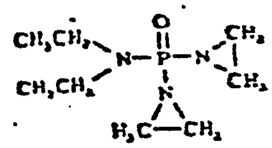

(LV)

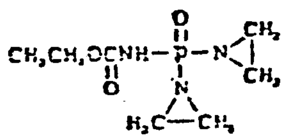

(LVI)

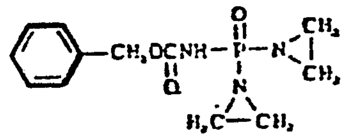

(LVII)

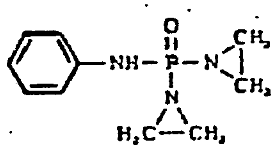

(LVIII)

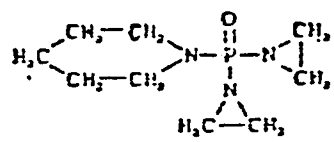

(LIX)

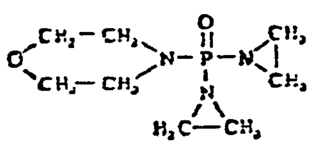

(LX)

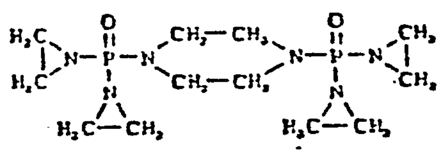

(LXI)

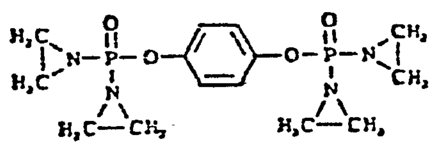

(LXII)

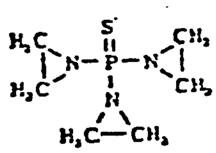

(LXIII)

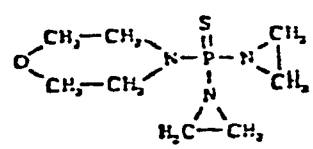

(LXIV)

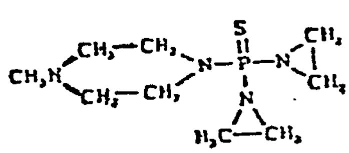

(LXV)

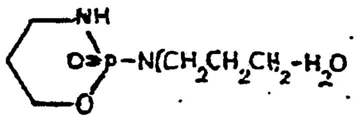
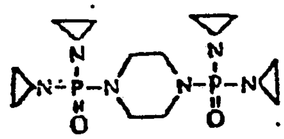
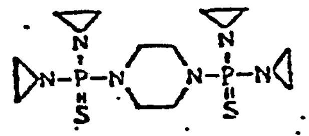

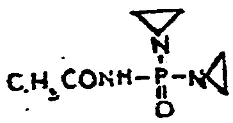
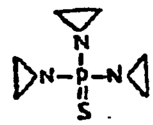
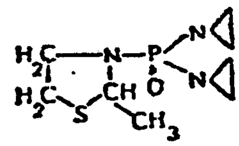

Stickstofflost, Cyclophosphamide, Triamcichon, Chlorambucil, Busulfan,

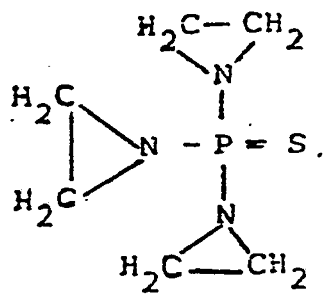
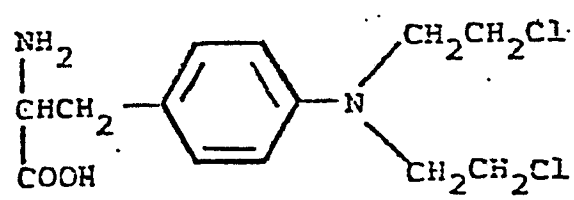

Nitomin, Mannitol-Stickstofflost, Amethopterin, 6-Mercaptopurin, 5-Fluorouracil, Cytosin-Arabinosid, Podohyllin, Actinomycin C, Actinomycin D, Mithramycin, Mitomycin C, Adriamycin, Bleomycin, Asparaginase, Ibenzmethycin,

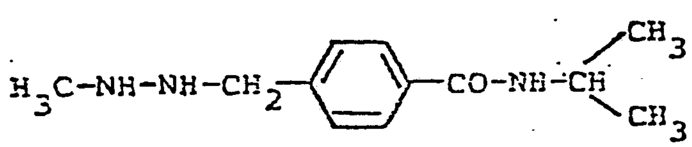
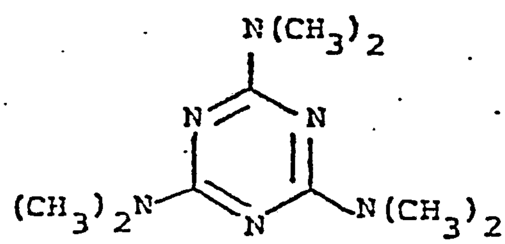

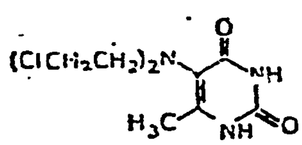
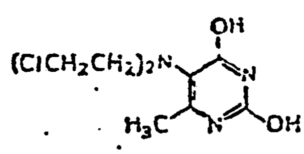
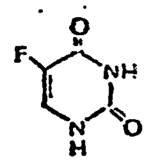
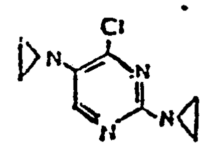

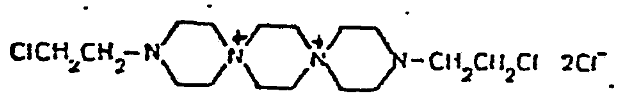
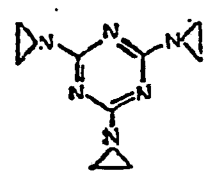

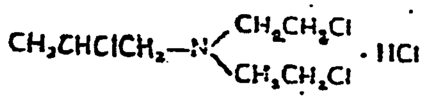

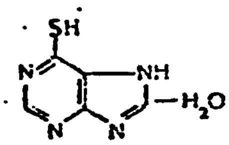

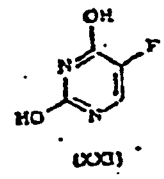

(XXI)

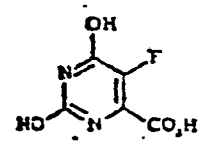

(XXII)

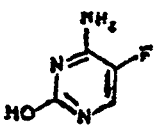

(XXIV)

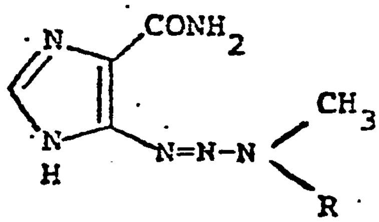

wobei R = H oder $CH_3$

Gegenstand der Erfindung ist nun die Verwendung der Stoffe der allgemeinen Formel (I),

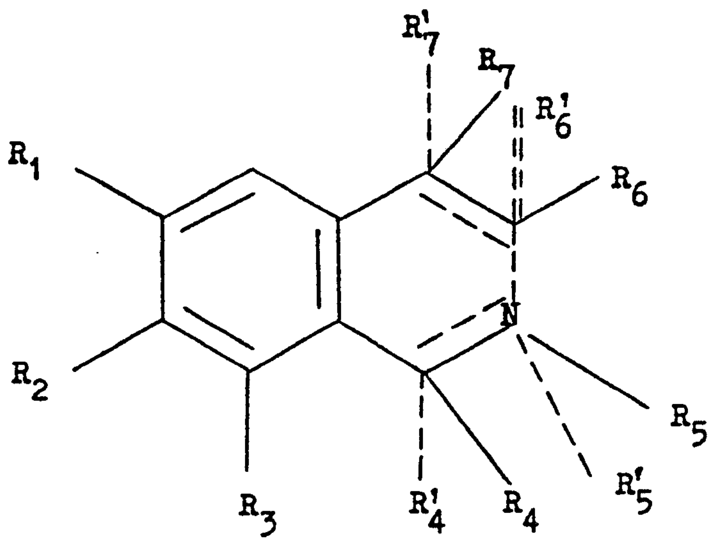

(I)

oder deren Salzen mit pharmazeutisch verwendbaren Säuren, worin bedeuten:

$R_1$ = H; $OCH_3$;

$R_2$ = $OCH_3$; OH; 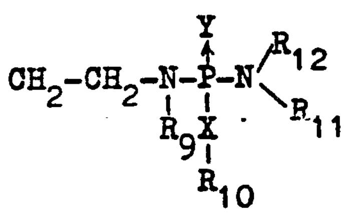 ;

oder $R_1$ und $R_2$ zusammen

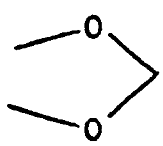

$R_3$ = H; $OCH_3$; 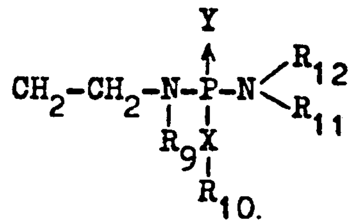

oder $R_2$ und $R_3$ zusammen

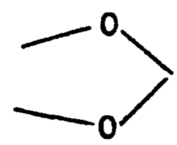

$R_4$ = H;
$R'_4$ = H;
oder $R_4 + R'_4 = 0$
oder $R_3$ und $R_4$ zusammen

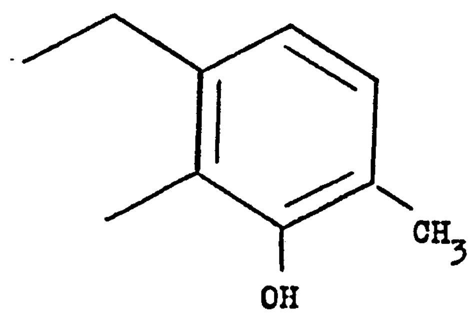
;

$R_5$ = $CH_3$; OH; 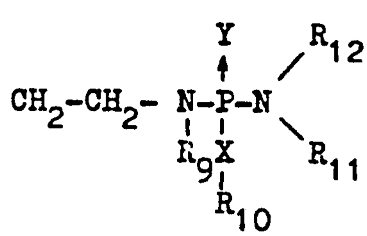
;

$R'_5$ = $CH_3$; OH;

oder $R_4$ und $H_5$ zusammen

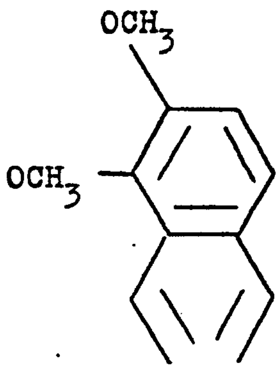 ; 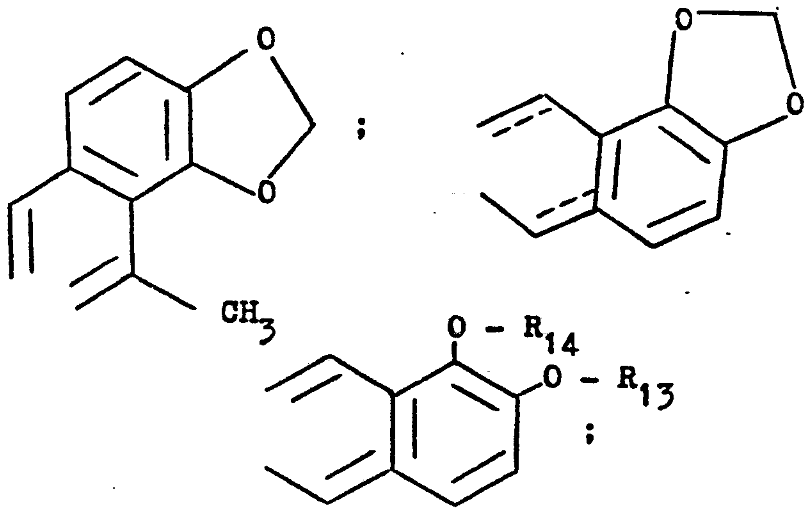 ; ; ;

$R_6 = H$;
$R'_6 = H$;
oder zusammen mit $R_6 = 0$;
oder $R_5$ und $R_6$ zusammen

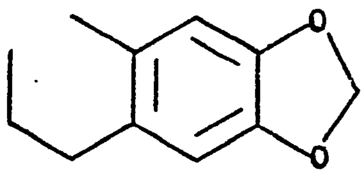 ; 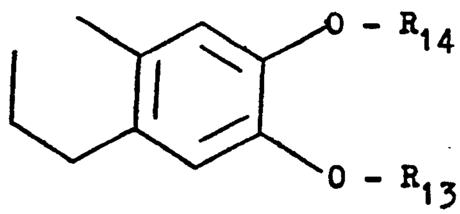 ,

mit der Maßgabe, daß wenigstens einer der Reste $R_2$, $R_3$ und $R_5$ (allein oder zusammen mit $R_4$ oder $R_6$) eine

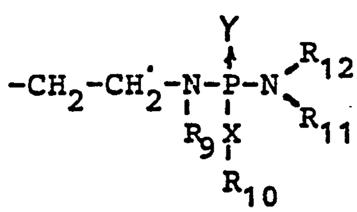 Gruppe ist bzw. enthält,

$R_7 = H$;
$R'_7 = H$;
oder $R_{6d}$ und $R_7$ zusammen

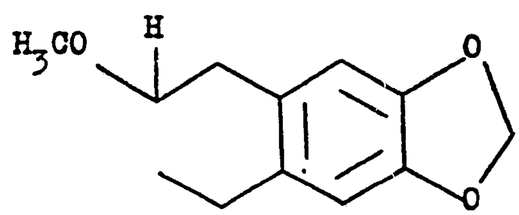 ; 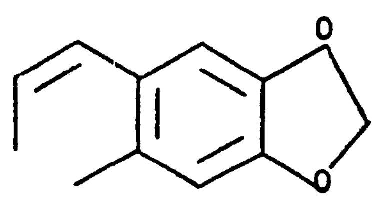 ;

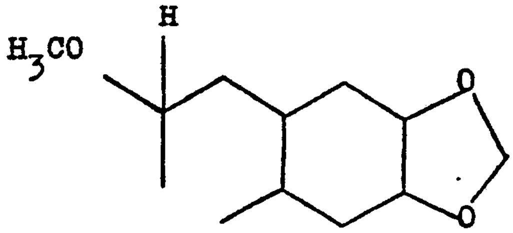 ; 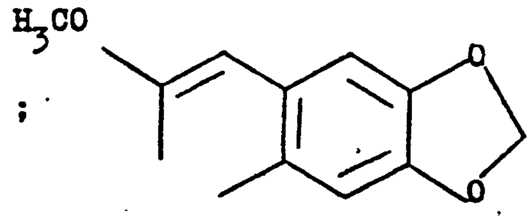 ;

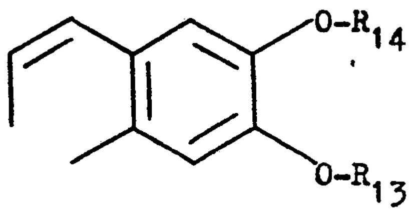 ; 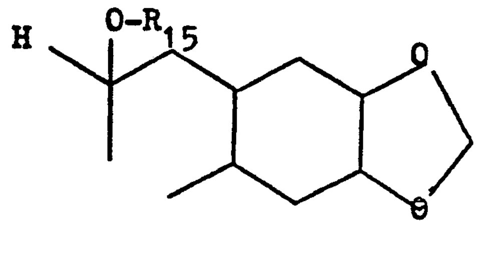 ;

$R_9$ = H; $CH_2$-$CH_2$-Cl; Alkaloide:
$R_{10}$ = H; OH; Alkaloid;
$R_{11}$ ; H; Alkaloid;
oder
$R_{11}$ + $R_{12}$ = $CH_2$ - $CH_2$;,
Y = S; 0;
X = NH; 0;
$R_{12}$ = H; Alkaloid;,

$R_{13}$ = OH; 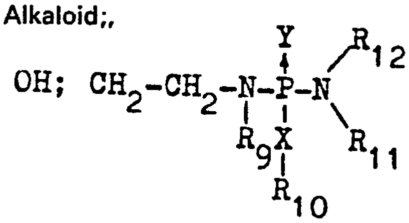 ;

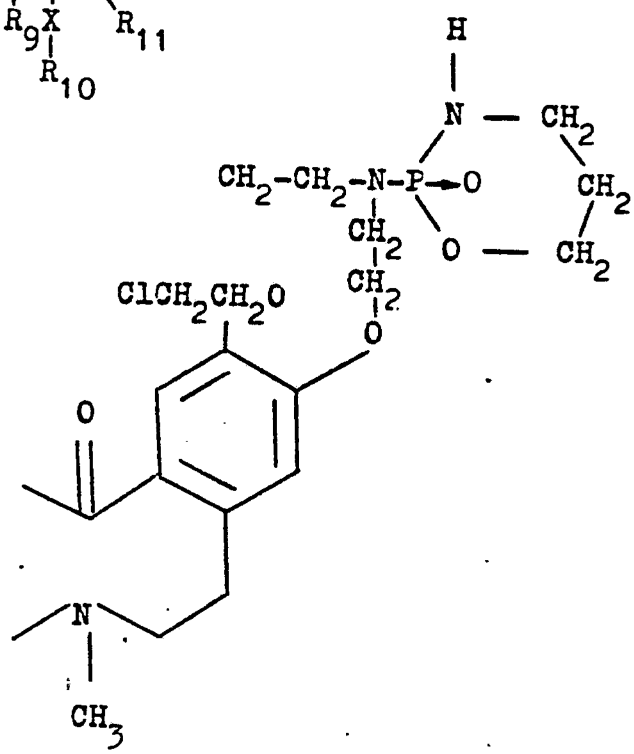 ;

$R_{14}$ = OH; $CH_2$-Cl; 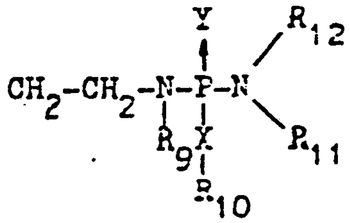 ;

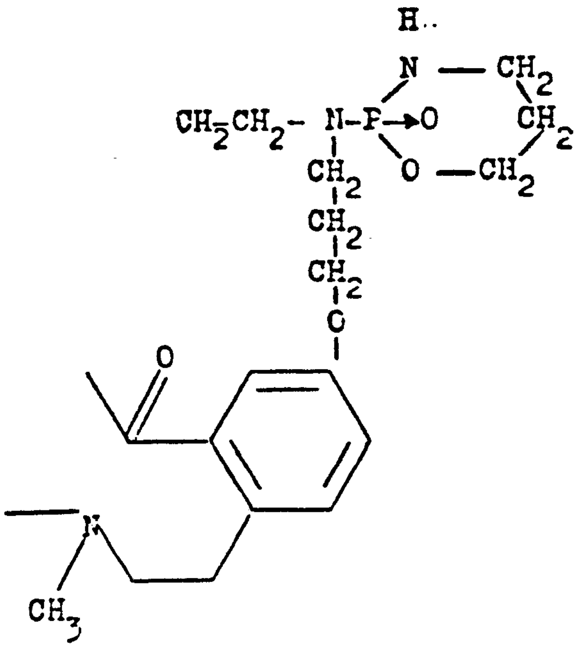 ;

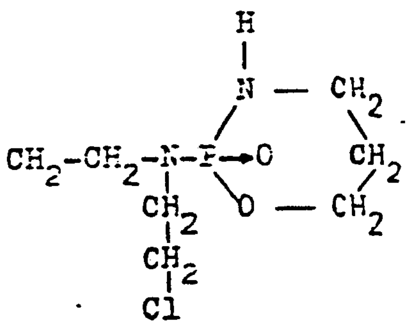 ;

$R_{15}$ = 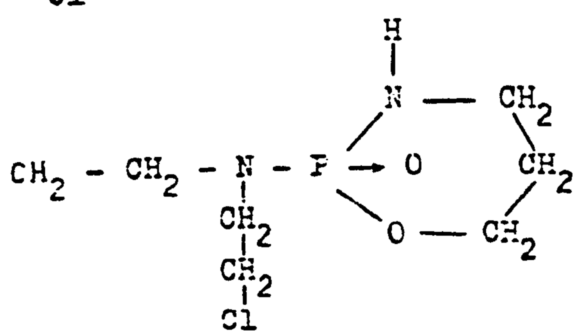 ;

zur Herstellung eines Arzneimittels zur Stärkung des Immunsystems und zur Bekämpfung von Infektionskrankheiten, insbesondere zur Behandlung von viralen, bakteriellen und Pilz-Infektionen.

Die Salzbildung kann praktisch mit jeder pharmazeutisch unbedenklichen Säure, die an sich hinreichend wasserlöslich ist und damit hinreichend wasserlösliche Salze liefert, durchgeführt werden; aus ökonomischen Gründen wird vorzugsweise Salzsäure eingesetzt, wodurch die entsprechenden Hydrochloride entstehen.

Die erhaltenen Alkaloidthiophosphorsäureamid-Salze unterscheiden sich in ihrer pharmakologischen Wirksamkeit nicht von den entsprechenden Basen; sie sind jedoch im Hinblick auf ihre wesentlich bessere Wasserlöslichkeit leichter und exakter zu dosieren, und es treten keine, den bisher notwendigerweise verwendeten organischen Lösungsmitteln zuzuschreibende störende Nebeneffekte auf.

Die Umsetzung der Alkaloidsalze wird zweckmäßig in einem Lösungsmittel bzw. Lösungsmittelgemisch bei erhöhter Temperatur durchgeführt; es kann aber auch zunächst die Alkaloidbase mit dem Thiophosphorsäureamid umgesetzt werden, worauf das Reaktionsprodukt in das Salz übergeführt werden kann. Die Umsetzung des Reaktionsproduktes mit der jeweils gewünschten Säure wird zweckmäßig in einem organischen Lösungsmittel durchgeführt, wobei nach Salzbildung das jeweilige Salz ausfällt bzw. durch Ausschütteln mit Wasser oder wässeriger Säure in die wässerige Lösung extrahiert werden kann.

Die Zusammensetzung eines Präparates, beispielsweise jenes, das aus Alkaloiden des Chelidonium majus L. besteht, basiert auf der Reaktion der Alkaloide mit alkylierenden Substanzen, etwa mit Thiophosphorsäuretriaziridid (Thio-TEPA).

Diese Substanz hat drei reaktive Gruppen, die sich entweder mit den Alkaloidmolekülen verbinden oder durch OH oder $NH_2$-Gruppen substituiert werden können. Auf diese Weise können, wenn sich diese Substanz mit einem einzelnen, reinen Alkaloid verbindet, eine ganze Reihe von verschiedenen Reaktionsprodukten entstehen. Um nun einfachere Reaktionen zu untersuchen, wurden in Versuchsreihen einzelne Alkaloide mit Thio-TEPA, Cyclophosphamid und anderen organischen Verbindungen versetzt. Im Falle des Chelidonins ließen sich mindestens 12 Reaktionsprodukte durch Dünnschichtchromatographie nachweisen. Um eine exakte Analyse dieser Reaktionsprodukte zu erhalten, wurden einige chromatographisch isoliert auskristallisiert.

Die sich bei der Elementaranalyse dieser Komponenten ergebenden chemischen Formeln sind in ihrer möglichen molekularen Struktur auf den Abbildungen 1 bis 17 dargestellt.

Wenn eine Mischung verschiedener Alkaloide mit Thi-TEPA versetzt wird, besteht die Möglichkeit, daß drei Moleküle verschiedener Alkaloide über ein Thio-TEPA-Molekül miteinander verbunden sind und daher eine sehr große Anzahl von Komponenten synthetisiert wird. In dieser sehr komplexem Mischung aus Reaktionsprodukten sind einige enthalten, welche für die einzigartige Form von biologischer Aktivität verantwortlich sein können.

Tatsächlich ergaben sich bei Auftrennung der Reaktionsprodukte von Thio-TEPA und dem Alkaloidextrakt von Chelidonium majus L. mit Hilfe der zweidimensionalen Dünnschichtchromatographie mehr als 50 im UV-Licht fluoreszierende Punkte.

Weiterhin ließ sich nachweisen, daß in dem Präparat Ukrain (hier erfolgte die Umsetzung der Alkaloide von Chelidonium majus L. mit Thiophosphorsäuretriaziridid, gen. Thio-TEPA) keine freien Äthylengruppen des Thio-TEPA vorliegen. Dieser Nachweis wurde erbracht:

1. durch eine chemische Analyse und
2. dadurch, daß beim biologischen Test die Dosis letalis für Thio-TEPA bei 1 mg/kg Körpergewicht liegt, für das Präparat jedoch eine Dosis von 250 mg/kg Körpergewicht nicht toxisch ist.

Außerdem hat das Präparat keine Wirkung auf Leukämie 1210 und keine auf das Blutbild. Daraus läßt sich ableiten, daß die Äthylengruppen des Thio-TEPA, die auch für seine toxische Wirkung verantwortlich sind, durch die Alkaloide blockiert sind.

Die Abbildungen zeigen wie vorgenannt 17 verschiedene chemische Strukturen, die sich aus der vollständigen Analyse einiger Reaktionsprodukte von Thio-TEPA und Cyclophosphamid mit Reinalkaloiden ergeben haben.

Abb. 1 Chelilutin + Thiophosphorsäuretriaziridid (1-Chelilutin, 2-Thiophosphorsäuretriaziridid, 3-Chelilutin + Thiophosphorsäuretriaziridid).

| | | |
|---|---|---|
| Berechnet: | C = 49,44 % ; | H = 6,36 % ; |
| | N = 11,53 % ; | P = 8,49 % ; |
| gefunden: | C = 49,41 % ; | H = 6,34 % ; |
| | N = 10,65 % ; | P = 8,67 % ; |

Abb. 2 Chelerythrin + Thiophosphorsäuretriaziridid $C_{66}H_{69}N_6O_{15}PS$.

| | | |
|---|---|---|
| Berechnet: | C = 63,45 % ; | H = 5,56 % ; |
| | N = 6,72 % ; | P = 2,47 % ; |
| gefunden: | C = 62,69 % ; | H = 5,37 % ; |
| | N = 5,37 % ; | P = 2,35 % ; |

Abb. 3 Coptisin + Thiophosphorsäuretriaziridid $C_{24}H_{27}N_4U_5PS$.

| | | |
|---|---|---|
| Berechnet: | C = 56,02 % ; | H = 5,28 % ; |
| | N = 10,88 % ; | P = 6,01 % ; |
| | S = 6,23 % ; | |
| gefunden: | C = 55,94 % ; | H = 5,12 % ; |
| | N = 11,10 % ; | P = 5,89 % ; |
| | S = 6,10 % ; | |

Abb. 4 Chelidonin + Thiophorsäuretriaziridid $C_{66}H_{75}N_6O_{18}PS$.

| | | | |
|---|---|---|---|
| Berechnet: | C = 60,82 % ; | H = 5,79 % ; | N = 6,44 % ; |
| | P = 2,37 % ; | S = 2,45 % ; | |
| gefunden: | C = 61,14 % ; | H = 5,76 % ; | N = 5,94 % ; |
| | P = 2,40 % ; | S = 2,39 % ; | |

Abb. 5 Protopin + Thiophosphorsäuretriaziridid $C_{32}H_{55}N_{11}O_3P_4S_3$.

| | | | |
|---|---|---|---|
| Berechnet: | C = 44,59 % ; | H = 6,43 % ; | N = 17,87 % ; |
| | P = 14,37 % ; | S = 11,16 % ; | |
| gefunden | C = 44,58 % ; | H = 6,14 % ; | N = 17,76 % ; |
| | C = 44,72 % ; | H = 6,30 % ; | N = 17,77 % ; |
| | C = 14,04 % ; | S = 12,71 % ; | |

Abb. 6 Reserpine + Thiophophosphoric Triaziridide $C_{72}H_{92}N_7PSO_{18}:2H_2O$, mp 120-125°

| | | | | |
|---|---|---|---|---|
| Berechnet: | C = 59,94 % ; | H = 6,70 % ; | N = 6,79 % ; | S = 2,22 % ; |
| gefunden: | C = 59,89 % ; | H = 6,62 % ; | N = 6,82 % ; | S = 2,26 % ; |

Abb. 7 Reserpine + Thiophosphoric Triaziridide $C_{35}H_{46}N_3O_{11}PS$.

| | | | | |
|---|---|---|---|---|
| Berechnet: | C = 56,21 % ; | H = 6,20 % ; | N = 5,61 % ; | P = 4,14 % ; |
| | S = 4,28 % ; | | | |
| gefunden: | C = 56,30 % ; | H = 6,22 % ; | N = 4,11 % ; | |

Abb. 8 Coffein + Thiophosphoric Triaziridide $C_{30}H_{42}N_{15}PSO_6$, mp 110-112° ; 215-216°

| | | | | |
|---|---|---|---|---|
| Berechnet: | C = 46,68 % ; | H = 5,48 % ; | N = 27,22 % ; | P = 4,01 % ; |
| | S = 4,15 % ; | | | |
| gefunden: | C = 47,37 % ; | H = 5,44 % ; | N = 27,25 % ; | P = 4,02 % ; |
| | S = 4,15 % ; | | | |

Abb. 9 Narcotine + Thiophosphoric Triaziridide $C_{28}H_{35}N_4PSO_7$, mp 225-226°

| | | | | | |
|---|---|---|---|---|---|
| Berechnet: | C=55,80%; | H=5,85%; | N=9,29%; | P=5,13%; | S=5,29%; |
| gefunden: | C=55,34%; | H=5,69%; | N=9,52%; | P=4,80%; | S=5,29%; |

Abb. 10 Aconitine + Thiophosphoric Triaziridide $C_{74}H_{106}N_5O_{22}PS$, mp 197-200°

| | | | | | |
|---|---|---|---|---|---|
| Berechnet: | C=60,02%; | H=7,21%; | N=4,72%; | P=2,09%; | S=2,16%; |
| gefunden: | C=60,30%; | H=7,22%; | N=4,38%; | P=2,09%; | S=2,16%; |

Abb. 11 Aconitine + Thiophosphoric Triaziridide $C_{39}H_{59}N_4O_{11}PS$, mp 210-211°

| | | | | | |
|---|---|---|---|---|---|
| Berechnet: | C=56,92%; | H=7,22%; | N=6,80%; | P=3,76%; | S=3,86%; |
| gefunden: | C=56,91%; | H=7,12%; | N=6,89%; | P=3,60%; | S=3,73%; |

Abb. 12 Aconitine + Thiophosphoric Triaziridide $C_{35}H_{55}N_4PSO_{11}$, mp 190-192°

| | | | |
|---|---|---|---|
| Berechnet: | C=54,83%; | H=7,19%; | N=7,26%; |
| gefunden: | C=54,83%; | H=6,98%; | N=8,74%; |

Abb. 13 Pilocarpine + Thiophosphoric Triaziridide $C_{26}H_{31}N_3O_7$

Abb. 14 Allocryptopine + Cyclophosphamide (N,N-Bis-(ß-chloräthyl)-N',O-Propylenphosphorsäureesterdiamid) $C_{49}H_{65}N_4O_{12}Cl_2P$, mp 159-160°

| | | | | | |
|---|---|---|---|---|---|
| Berechnet: | C=58,22%; | H=6,27%; | N=5,58%; | P=3,08%; | Cl=7,06%; |
| gefunden: | C=58,25%; | H=6,25%; | N=5,40%; | P=2,53%; | Cl=7,41%; |
| | C=54,84%; | H=6,16%; | N=5,62%; | P=2,51%; | Cl=7,26%; |

Abb. 15 Protopine + Cyclophosphamide (N,N-Bis-(ß-chloräthyl)-N',O-Propylenphosphorsäureesterdiamid) $C_{47}H_{55}N_4O_{12}PCl_4$, mp 239-242°

| | | | | | |
|---|---|---|---|---|---|
| Berechnet: | C=54.24%; | H=5,32%; | N=5,38%; | P=2,97%; | Cl=13,62%; |
| gefunden: | C=54.04%; | H=5,25%; | N=4,85%; | P=2,72%; | Cl=10,13%; |
| | C=54,48 %; | H=5,22%; | N=4,69%; | P= | Cl=9,91%; |

Abb 15a Protopine + Cyclophosphamide (N,N-Bis-(ß-chloräthyl)-N',O-propylenphosphorsäureesterdiamid) $C_{47}H_{59}N_4O_{12}PCl_4$ mp 239-242°

| | | | | | |
|---|---|---|---|---|---|
| Berechnet: | C=54,03%; | H=5,69%; | N=5,36%; | P=2,96%; | Cl=13,57%; |
| gefunden: | C=54,04%; | H=5,25%; | N=4,85%; | P=2,27%; | Cl=10,13%; |
| | C=54,48%; | H=5,22%; | N=4,69%; | | Cl=9,91%; |

Abb. 15b Protopine + Cyclophosphamide (N,N-Bis-(ß-chloräthyl)-N',O-propylenphosphorsäureesterdiamid) $C_{47}H_{59}N_4O_{12}PCl_4$, mp 239-242°

| | | | | | |
|---|---|---|---|---|---|
| Berechnet: | C=54,03%; | H=5,69%; | N=5,36%; | P=2,96%; | Cl=13,57%; |
| gefunden: | C=54,04%; | H=5,25%; | N=4,85%; | P=2,27%; | Cl=10,13%; |
| | C=54,48%; | H=5,22%; | N=4,69%; | | Cl=9,91%; |

Abb. 16 Chelerythrine + Cyclophosphamide (N,N-Bis-(3-chloräthyl)-N',O-propylenphosphorsäureesterdiamid) $C_{27}H_{37}N_3O_9PCl_2$, mp 188-192°

| | | | | | |
|---|---|---|---|---|---|
| Berechnet: | C=49,93%; | H=5,74%; | N=6,64%; | P=4,76%; | Cl=10,91%; |
| gefunden: | C=49,85%; | H=5,31%; | N=6,06%; | P=4,95%; | Cl=13,23%; |
| | C=49,84%; | H=5,24%; | N=5,96%; | | Cl=14,24%; |

Abb. 17 Chelidonine + Cyclophosphamide (N,N-Bis-(ß-chloräthyl)-N',O-propylenphosphorsäureesterdiamid) $C_{27}H_{35}N_3O_7PCl_3$, mp 273-276°

| | | | | | |
|---|---|---|---|---|---|
| Berchnet: | C=49,82%; | H=5,41%; | N=6,45%; | P=4,75%; | Cl=16,33%; |
| gefunden: | C=49,26%; | H=5,07%; | N=5,12%; | P=3,50%; | Cl=16,50%; |

Für diagnostische Untersuchungen ist von Bedeutung, daß das Präparat Ukrain (Chelidonium majus L. Alkaloid-Thiophosphorsäuretriaziridid-derivaten) die Eigenschaft hat, im ultravioletten Licht gelbgrünlich zu fluoreszieren.

Die Anregungsfrequenzen liegen innerhalb des Frequenzbereiches von 220 bis 490 nm. Die Spektralbreite der Fluoreszenz erstreckt sich von 410 nm bis 665 nm, das Maximum liegt bei 550 nm. Diese große Spektralbreite erklärt sich dadurch, daß sich das Präparat aus einer Gruppe verschiedener Alkaloidderivate zusammensetzt. Die Sichtbarkeitsgrenze der Fluoreszenzerscheinung unter UV 366 nm an der Platte für Dünnschichtchromatographie liegt bei einer Verdünnung von 0,000007 mg/ml, 0,0000003 mg pro 1 $mm^2$.

Diese Eigenschaft behält das Präparat auch im lebenden Körper bei, so daß in klinischen Versuchen auch die Verteilung des Präparates Ukrain im Organismum beobachtet werden konnte.

Es wurden Untersuchungen über die immunstimulierende Wirkung von Ukrain in vitro im sogenannten Lymphozyten-Transformations- Test durchgeführt. Lymphozyten sind bekanntlich immunkompetent, wenn sie fähig sind, das spezifische Antigen zu erkennen, oder wenn sie fähig sind, auf dieses zu reagieren. Solche Zellen werden Immunozyten genannt und ihre Vorstufen Immunoblasten.

Die Bildung von Immunoblasten kann auch in vitro durchgeführt werden. Die Immunoblasten haben eine Größe von 20-30µm, normale Lymphozyten dagegen nur eine Größe von 5-15 µm.

Um die biologische Wirkungsweise von Ukrain zu erforschen, wurden folgende immunologische Untersuchungen durchgeführt. Für die Blastentransformation wurden isolierte Lymphozyten von Meerschweinchen nach Zusatz von Ukrain verwendet. Für die Isolation wurde eine Mischung der unter dem Handelsnamen Ficoll 4000 erhältlichen Verbindung EDTA benutzt (0,9 EDTA + 0,1 Ficoll 4000).

Die isolierten Lymphozyten wurden in vitro in Parker-Lösung mit einem Zusatz von 1,6 µg, 0,16 µg und 0,016 µg gezüchtet. Gleichzeitig wurden Lymphozyten in Parker-Lösung mit dem unspezifischen Stimulator Phytohämaglutinin (PHA) 5 µg/ml und ohne Zusatz zur Kontrolle gezüchtet. Um eine Bakterienbediedelung zu vermeiden, wurde die nötige Menge der Antibiotika Penicillin, Streptomycin und Nystatin zugefügt. Die Kulturen wurden bei 37°C inkubiert und während 3 Tagen täglich die Zahl der transformierten Lymphozyten unter dem Mikroskop ausgezählt.

In jedem Präparat wurden 100 Lymphozyten ausgezählt und diejenigen, die größer als 15 µ bei menschlichen

und 20 μ bei den Meerschweinchen Lymphozyten waren, wurden als transformiert gewertet. In allen Fällen war vor Beginn der Züchtung die Zahl der transformierten Zellen nicht größer als 10,1 %. Die Tests wurden mit den Lymphozyten von 10 gesunden Menschen und 10 gesunden Meerschweinchen durchgeführt. Diese Ergebnisse weisen deutlich darauf hin, daß das Präparat Ukrain eine Lymphozytentransformation bewirkt.

Die Dosis von Ukrain wurde, nach folgender Überlegung gewählt: da bei einer Kur mit Ukrain die mittlere Einzeldosis 16 mg beträgt, mit jeweils 1/10000; 1/100000 und 1/1000000 der Menge dosiert. Die Ergebnisse sind in den nachstehenden Tabellen I und II zusammengefaßt:

**Tabelle I**

Procente der transformierten Meerschweinchen-Lymphosyten

| Nr. | Kultur - Medium mit Zusatz | Bebrütungszeit 24 h | 48 h | 72 h |
|---|---|---|---|---|
| 1. | 1,6 µg/ml Ukrain | 48,6 | 45,6 | 40,2 |
| | 0,16 µg/ml Ukrain | 42,3 | 29,3 | 28,5 |
| | 0,016 µg/ml Ukrain | 35,6 | 33,6 | 25,0 |
| | PHA | 32,2 | 28,3 | 27,9 |
| | Ohne Stimulator | 19,2 | 15,3 | 1,8 |
| 2. | 1,6 µg/ml Ukrain | 53,9 | 30,2 | 18,3 |
| | 0,16µg/ml Ukrain | 48,7 | 32,2 | 32,0 (?) |
| | O,016 µg/ml Ukrain | 27,3 | 30,2 | 18,5 |
| | PHA | 20,8 | 17,2 | 17,5 |
| | Ohne Stimulator | 13,3 | 12,2 | 19,2 (?) |
| 3. | 1,6 µg/ml Ukrain | 50,2 | 48,3 | 42,8 |
| | 0,16 µ/ml Ukrain | 49,3 | 51,1 | 39,9 |
| | 0,016 µg/ml Ukrain | 41,0 | 29,3 | 30,1 |
| | PHA | 32,5 | 29,8 | 22,3 |
| | Ohne Stimulator | 18,0 | 11,2 | 10,3 |
| 4. | 1,6 µg/ml Ukrain | 55,6 | 42,3 | |
| | 0,16 µg/ml Ukrain | 50,8 | 40,7 | 43,8 |
| | 0,016 µg/ml Ukrain | 41,2 | 38,5 | 39,5 |
| | PHA | 33,2 | 27,0 | 18,0 |
| | Ohne Stimulator | 18,2 | 18,0 | 16,2 |
| 5. | 1,6 µg/ml Ukrain | 51,3 | 43,5 | 31,5 |
| | 0,16 µg/ml Ukrain | 48,2 | 33,2 | 33,5 |
| | 0,016 µg/ml Ukrain | 39,9 | 31,5 | 30,2 |
| | PHA | 33,4 | 20,3 | 11,3 |
| | Ohne Stimulator | 12,5 | 12,5 | 8,4 |
| 6. | 1,6 µg/ml Ukrain | 58,5 | 57,9 | 41,8 |
| | 0,16 µg/ml Ukrain | 48,3 | 50,0 | 43,2 |
| | 0,016 µg/ml Ukrain | 40,1 | 35,2 | 30,0 |
| | PHA | 30,2 | 22,1 | 17,3 |
| | Ohne Stimulator | 19,0 | 18,2 | 13,5 |
| 7. | 1,6 µg/ml Ukrain | 43,3 | 41,2 | 39,8 |
| | 0,16 µg/ml Ukrain | 42,3 | 36,6 | 36,2 |
| | 0,016 µg/ml Ukrain | 41,2 | 37,2 | 35,5 |
| | PHA | 33,3 | 33,4 | 27,2 |
| | Ohne Stimulator | 15,1 | 16,1 | 13,2 |
| 8. | 1,6 µg/ml Ukrain | 48,7 | 45,1 | 40,3 |
| | 0,16 µg/ml Ukrain | 43,1 | 39,2 | 31,1 |
| | 0,016 µg/ml Ukrain | 35,6 | 29,8 | 24,2 |
| | PHA | 31,3 | 27,0 | 24,3 |
| | Ohne Stimulator | 12,2 | 10,1 | 10,5 |
| 9. | 1,6 µg/ml Ukrain | 53,3 | 51,5 | 41,2 |
| | 0,16 µg/ml Ukrain | 49,6 | 47,2 | 42,2 |
| | 0,016 µg/ml Ukrain | 39,2 | 36,7 | 34,6 |
| | PHA | 38,3 | 32,8 | 25,2 |
| | Ohne Stimulator | 18,1 | 16,7 | 11,3 |
| 10. | 1,6 µg/ml Ukrain | 56,2 | 53,6 | 40,7 |
| | 0,16 µg/ml Ukrain | 49,3 | 42,8 | 34,5 |
| | 0,016 µg/ml Ukrain | 39,8 | 37,3 | 28,3 |
| | PHA | 34,2 | 33,2 | 27,5 |
| | Ohne Stimulator | 12,0 | 10,2 | 8,4 |

**Tabelle II**

Prozente der transformierten menschlichen Lymphozyten

| Nr. | Kultur-Medium mit Zusatz | Bebrütungszeit 24 h | 48 h | 72 h |
|---|---|---|---|---|
| 1. | 1,6 µg/ml Ukrain | 47,2 | 43,4 | 41,2 |
| | 0,16 µg/ml Ukrain | 43,8 | 39,2 | 38,6 |
| | 0,016 µg/ml Ukrain | 39,6 | 36,7 | 35,2 |
| | PHA 5 µg/ml | 36,6 | 24,8 | 20,8 |
| | Ohne Stimulator | 11,1 | 9,2 | 9,0 |
| 2. | 1,6 µg/ml Ukrain | 49,9 | 41,2 | 37,2 |
| | 0,16 µg/ml Ukrain | 43,2 | 40,3 | 33,2 |
| | 0,016 µg/ml Ukrain | 38,6 | 36,2 | 26,8 |
| | PHA 5 µg/ml | 31,2 | 28,0 | 21,2 |
| | Ohne Stimulator | 18,4 | 14,1 | 8,4 |
| 3. | 1,6 µg/ml Ukrain | 19,1 | 18,8 | 16,2 |
| | 0,16 µg/ml Ukrain | 19,5 | 18,2 | 15,3 |
| | 0,016 µg/ml Ukrain | 14,2 | 15,3 | 13,2 |
| | PHA 5 µg/ml | 33,1 | 28,2 | 14,1 |
| | Ohne Stimulator | 10,7 | 9,3 | 15,3 |
| 4. | 1,6 µg/ml Ukrain | 43,9 | 42,8 | 39,4 |
| | 0,16 µg/ml Ukrain | 41,2 | 40,6 | 41,2 |
| | 0,016 µg/ml Ukrain | 33,6 | 32,1 | 31,2 |
| | PHA 5 µg/ml | 40,2 | 33,6 | 28,3 |
| | Ohne Stimulator | 13,6 | 12,3 | 10,8 |
| 5. | 1,6 µg/ml Ukrain | 53,3 | 51,5 | 47,8 |
| | 0.16 µg/ml Ukrain | 48,6 | 43,6 | 41,0 |
| | 0,016 µg/ml Ukrain | 37,2 | 37,8 | 36,2 |
| | PHA 5 µg/ml | 36,4 | 28,5 | 25,4 |
| | Ohne Stimulator | 15,3 | 14,4 | 12,8 |
| 6. | 1,6 µg/ml Ukrain | 55,5 | 53,4 | 51,5 |
| | 0,16 µg/ml Ukrain | 51,2 | 49,1 | 47,3 |
| | 0,016 µg/ml Ukrain | 43,2 | 36,2 | 36,0 |
| | PHA 5 µg/ml | 37,1 | 36,2 | 34,7 |
| | Ohne Stimulator | 19,3 | 18,4 | 16,5 |
| 7. | 1,6 µg/ml Ukrain | 48,6 | 46,8 | 42,5 |
| | 0,16 µg/ml Ukrain | 47,2 | 45,2 | 24,5 |
| | 0,016 µg/ml Ukrain | 39,8 | 32,0 | 21,2 |
| | PHA 5 vg/ml | 30,1 | 26,8 | 19,0 |
| | Ohne Stimulator | 14,7 | 13,1 | 8,5 |
| 8. | 1,6 µg/ml Ukrain | 52,3 | 37,4 | 21,8 |
| | 0,16 µg/ml Ukrain | 48,3 | 28,3 | 19,3 |
| | 0,016 µg/ml Ukrain | 41,2 | 19,4 | 17,4 |
| | PHA 5 µg/ml | 37,3 | 19,5 | 20,1 |
| | Ohne Stimulator | 9,0 | 8,3 | 8,2 |
| 9. | 1,6 µg/ml Ukrain | 47,2 | 32,8 | 29,1 |
| | 0,16 µg/ml Ukrain | 43,4 | 36,4 | 34,2 |
| | 0,016 µg/ml Ukrain | 38,6 | 25,0 | 20,0 |
| | PHA 5 µg/ml | 36,6 | 28,6 | 21,4 |
| | Ohne Stimulator | 15,3 | 13,3 | 10,7 |
| 10. | 1,6 µg/ml Ukrain | 48,3 | 37,4 | 33,3 |
| | 0,16 µg/ml Ukrain | 43,4 | 41,2 | 36,8 |
| | 0,016 µg/ml Ukrain | 37,1 | 36,2 | 29,1 |
| | PHA 5 µg/ml Ukrain | 30,0 | 19,4 | 18,4 |
| | Ohne Stimulator | 9,3 | 9,6 | 5,3 |

In dem Test zeigte sich eine statistische signifikante Differenz zwischen der Anzahl der transformierten Lymphozyten von Menschen und Tieren und den Kontrollgruppen bei Anwesenheit von Ukrain. Daraus kann der Schluß gezogen werden, daß das Präparat Ukrain eine immunologische Wirkung besitzt und das menschliche Abwehrsystem stimuliert.

Nur in einem von 10 Fällen (siehe Tabelle II, Nr.3) zeigte sich keine deutliche Transformierung von Lymphozyten. Es handelt sich dabei um menschliche Lymphozyten. Bei Patient Nr. 3 könnte also unter Umständen ein "In vitro-Äquivalent" zu dem klinischen Phänomen vorliegen, bei dem einzelne Patienten auf

Ukrain überhaupt keine Reaktion zeigten, obwohl bei allen anderen Patienten eine höhere Transformationsrate erzielt wurde als mit PHA.

Es ist in diesem Zusammenhang besonders auffällig, daß bei sehr vielen Patienten nach den ersten Ukrain-Injektionen Infektionskrankheiten verschwanden; darunter waren hartnäckige Mycosen, die vorher auf keine Therapie angesprochen hatten, Fälle von chronischer Bronchitis und Halsentzündungen unklarer Genese, sowie andere, nach Ansicht der behandelnden Ärzte, sowohl virale als auch bakterielle Erkrankungen. Offenbar verursacht das Präparat eine Stimulierung der natürlichen Killerzellen, deren Funktion die Zerstörung von virsubefallenen Zellen bzw. Bakterien und Pilzen ist.

Schließlich ergab noch die Durchführung der an sich bekannten Agargelelektrophorese mit Ukrain unter Verwendung von 0,05 mg Ukrain, das auf Agargel (Sörensen Puffer + NaCl) in eine kleine Kerbe gegeben und 4 Stunden bei 70 Volt und 2mA gefahren wurde, wobei ein Filterpapier auf die Oberfläche gepreßt, der Start und die Polung markiert, bei Betrachtung unter der UV-Lampe ein zum negativen Pol gerichtetes fluoreszierendes Band. Die fluoreszierenden Fraktionen waren positiv geladen. Aus dem Vorstehenden wird klar erkennbar, daß die eingangs angesprochenen Präparate bzw. Verbindungen sowohl zum Diagnostizieren als auch zur therapeutischen Behandlung von infektiösen Krankheiten eingesetzt werden können, wofür die fluoreszierende Wirkung der Präparate von großer Bedeutung ist. Zur Diagnose ist es für die Früherkennung somit möglich, nach Injektion des bzw. der Präparate durch deren Kumulierung im erkrankten Gewebereich eine rasche und zuverlässige Frühdiagnose vorzunehmen, die sich erkennen läßt einmal in dem schnellen Abbau der Injektionsflüssigkeit aus dem injizierten Bereich und in der Lokalisierung derselben im erkrankten Bereich.

Diese Beobachtung erstreckt sich auch auf alle endoskopischen Verfahren wie Bronchoskopie, Mediastinoskopie, Thorakoskopie, Oesophagoskopie, Oto-Rhinoskopie, Laryngoskopie, Rektoskopie, Proktoskopie u.a., wobei z. B. Kaltlicht durch UV-Licht ersetzt wird.

In diesem Zusammenhang ist als weitere diagnostische Methode denkbar die Anlagerung eines radioaktiven Isotops an das Präparat, so daß in an sich bekannter Weise die Radioaktivität mit entsprechenden Detektoren gemessen und ihre Kumulierung beispielsweise mit einem Gamma-Strahlen-Scanner aufgezeichnet werden kann. Die vorstehenden Ergebnisse zeigen aber auch die Stimulierung etwa der Lymphozyten zu Immunozyten mittels der aufgezeigten Präparate, was gleichbedeutend ist mit der Erzeugung der sogenannten Killer-Zellen, die das befallene bzw. körperfremde Gewebe oder die eingedrungenen Viren und dgl. zerstören.

Schließlich ist neben der Markierung der aufgezeigten Präparate mit fluoreszierenden Stoffen und radioaktiven Isotopen auch noch eine Dotierung mit strahlungsabsorbierenden Verbindungen oder Radikalen denkbar, so daß etwa die Kumulierung in einem bestimmten Gewebebereich durch die dann dort erhöhte Absorption von Röntgenstrahlung erkennbar wird.

**Patentanspruch**

Verwendung der Stoffe der allgemeinen Formel (I)

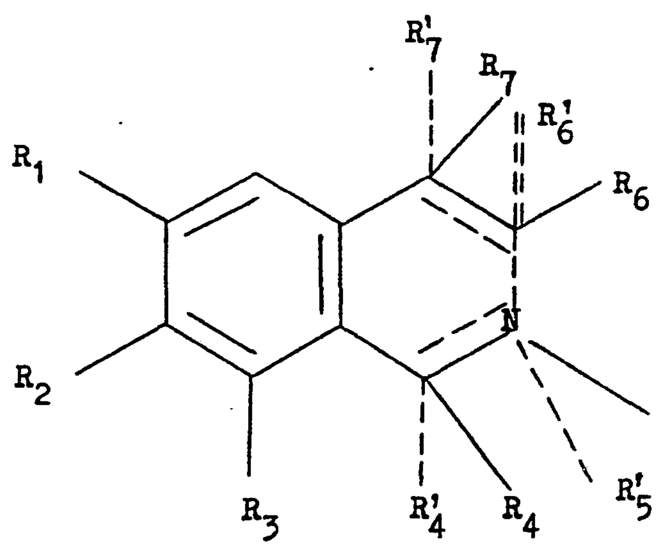

(I)

oder deren Salzen mit pharmazeutisch verwendbaren Säuren, worin bedeuten:

$R_1 = H; OCH_3;$

$R_2 = OCH_3$; OH; 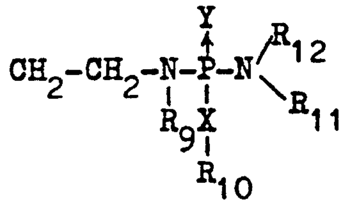 ;

oder $R_1$ und $R_2$ zusammen

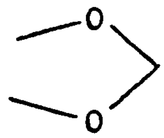

$R_3 = H$; $OCH_3$; 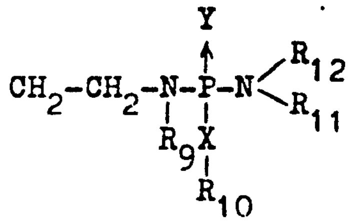

oder $R_2$ und $R_3$ zusammen

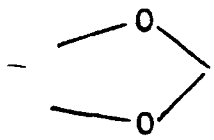

$R_4 = H$;
$R'_4 = H$;
oder $R_4 + R'_4 = 0$
oder $R_3$ und $R_4$ zusammen

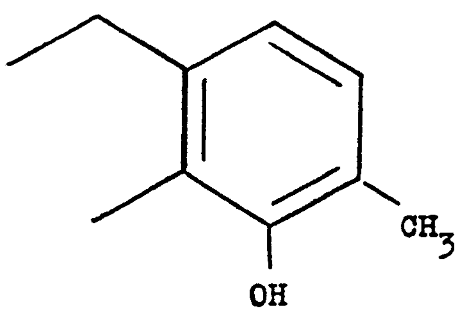

;

$R_5 = CH_3$; OH; 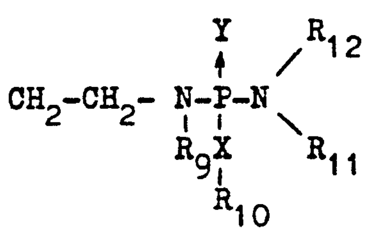 ;

$R'_5 = CH_3$; OH;
oder $R_4$ und $R_5$ zusammen

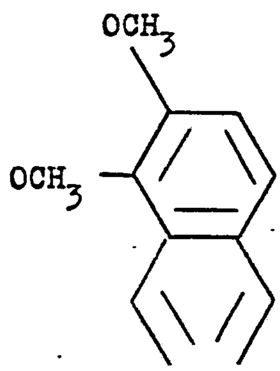 ; 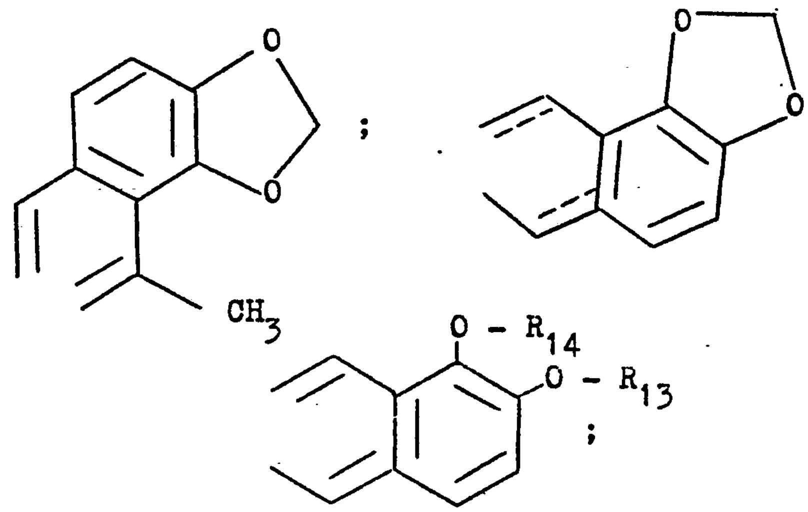 ; ; ;

$R_6 = H$;
$R'_6 = H$; oder zusammen mit $R^6 = O$;
oder $R_5$ und $R_6$ zusammen

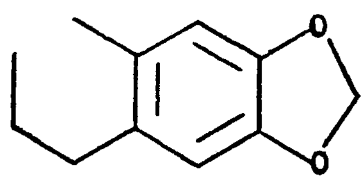 ; 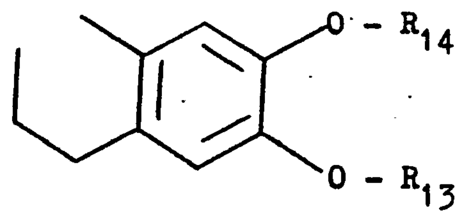 ,

mit der Maßgabe, daß wenigstens einer der Reste $R_2$, $R_3$ und $R_5$ (allein oder zusammen mit $R_4$ oder $R_6$) eine

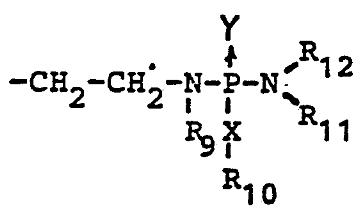 Gruppe ist bzw. enthält,

$R_7$ = H;
$R'_7$ = H;
oder $R_6$ und $R_7$ zusammen

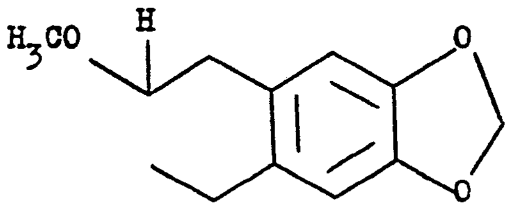 ; 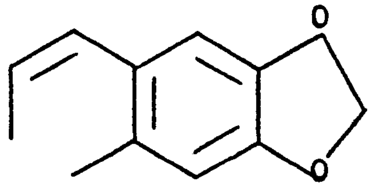 ;

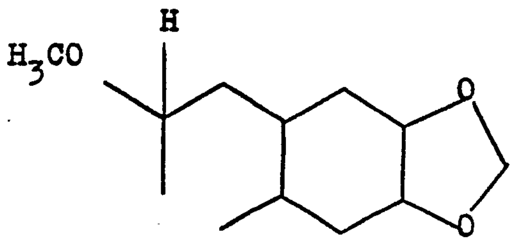 ; 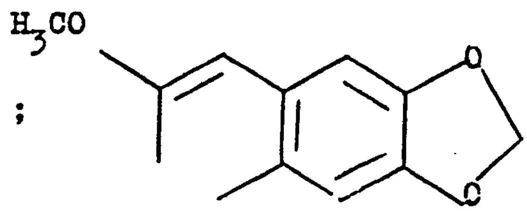 ;

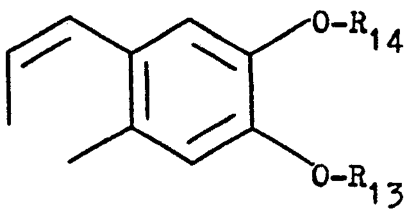 ; 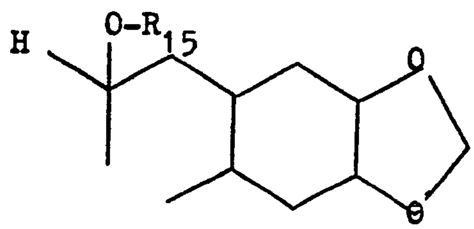 ;

$R_9$ = H; $CH_2$-$CH_2$-Cl; Alkaloide:
$R_{10}$ = H; OH; Alkaloid;
$R_{11}$ = H; Alkaloid;
oder
$R_{11}$ + $R_{12}$ = $CH_2$ - $CH_2$;
Y = S; O;
X = NH; O;
$R_{12}$ = H; Alkaloid;

$R_{13}$ = OH; 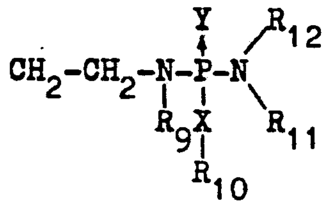 ;

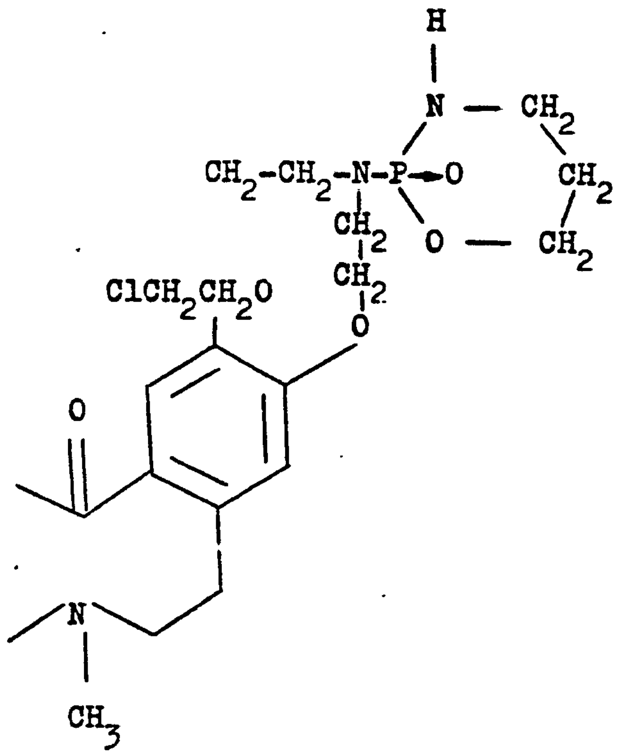 ;

$R_{14}$ = OH; $CH_2$-Cl; 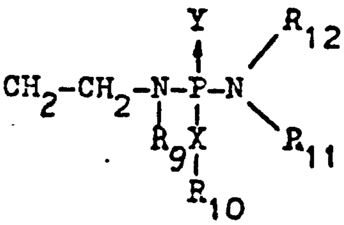 ;

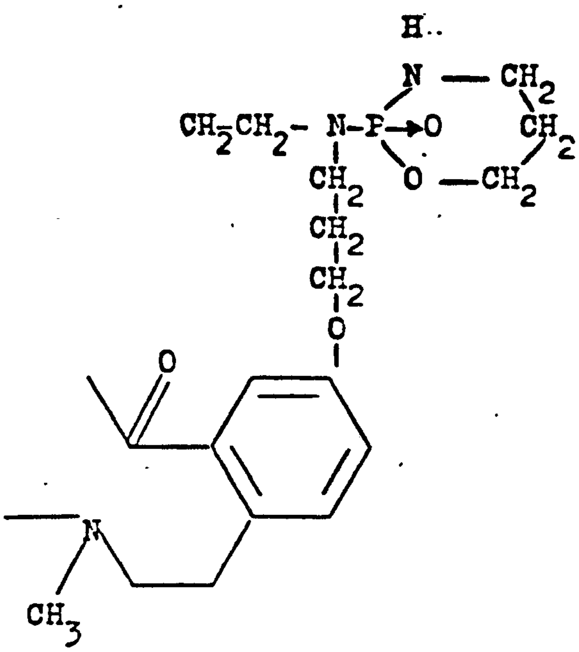 ;

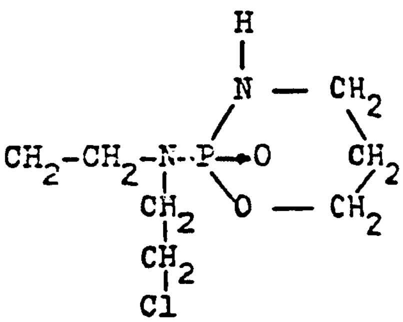 ;

$R_{15}$ = 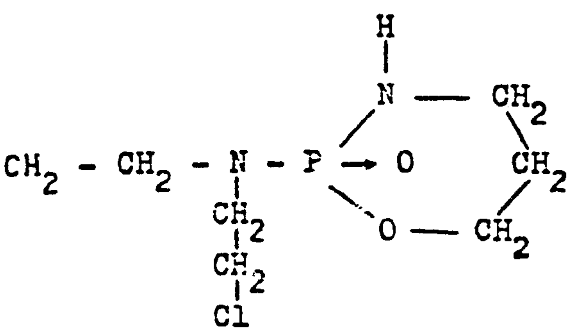 ;

zur Herstellung eines Arzneimittels zur Stärkung des Immunsystems des menschlichen und tierischen Körpers und zur Bekämpfung von Infektionskrankheiten, insbesondere zur Behandlung von viralen, bakteriellen und Pilz-Infektionen.

**Claim**

Use of substances with the general formula (I)

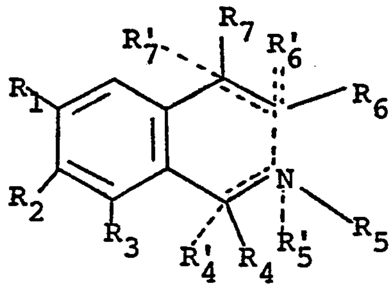

(I)

or their salts with pharmaceutically usable acids, where:
$R_1$ = H; $OCH_3$

$R_2$ = $OCH_3$; OH; 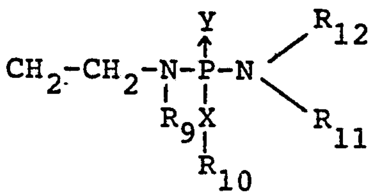 ;

or $R_1$ and $R_2$ together

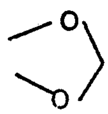

$R_3$ = H; $OCH_3$; 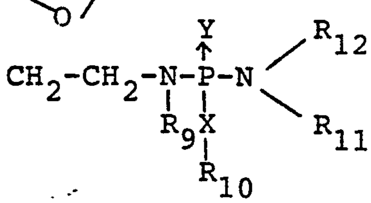

or $R_2$ and$R_3$ together

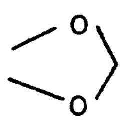

$R_4$ = H;
$R'_4$ = H;
or $R_4 + R^1_4 = O$
or $R_3$ and $R_4$ together

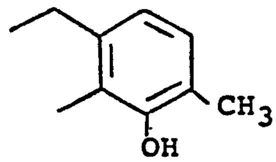

$R_5$ = $CH_3$; OH; 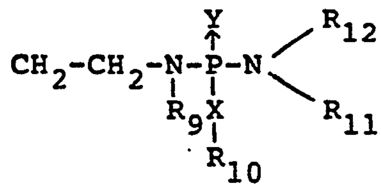 ;

$R'_5$ = $CH_3$; OH;
or $R_4$ and $R_5$ together

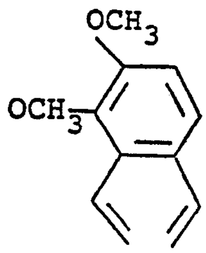 ; 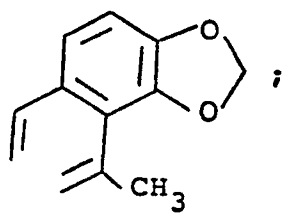 ; 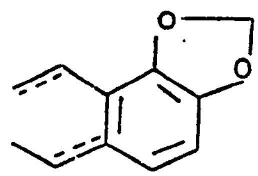 ;

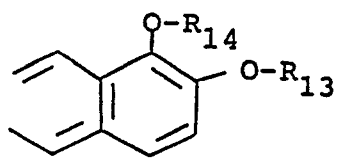 ;

$R_6$ = H;
$R'_6$ = H; or together with $R_6$ = O;
or $R_5$ and $R_6$ together

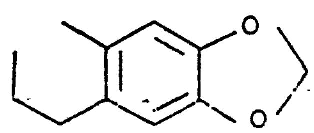 ; 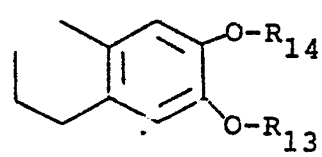

with the provision that at least one of the remaining $R_2$, $R_3$ or $R_5$ (on its own or with $R_4$ or $R_6$ ) is or contains a

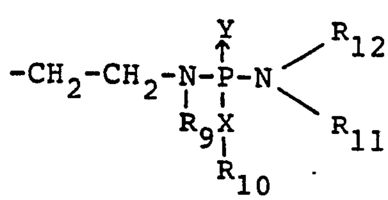 group,

$R_7$ = H;
$R'_7$ = H;
or $R_6$ and $R_7$ together

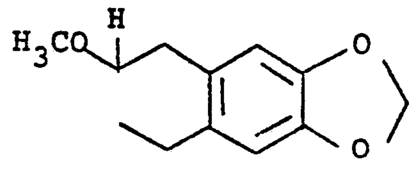 ; 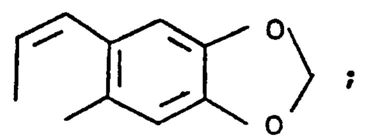 ;

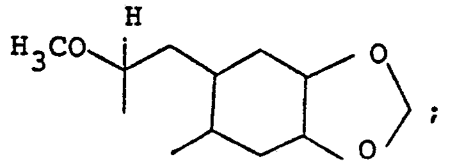 ; 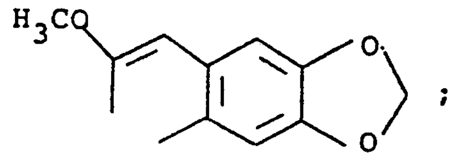 ;

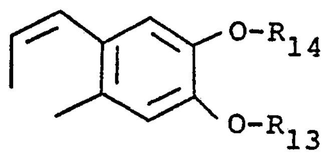 ; 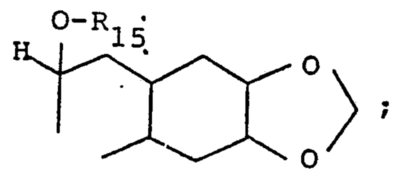 ;

$R_9$ = H; $CH_2$-$CH_2$-Cl; alkaloids:
$R_{10}$ = H; OH; alkaloid;
$R_{11}$ = H; alkaloid;
or
$R_{11}$ + $R_{12}$ = $CH_2$-$CH_2$;
Y = S; O;
X = NH; O;
$R_{12}$ = H; alkaloid;

$R_{13}$ = OH; 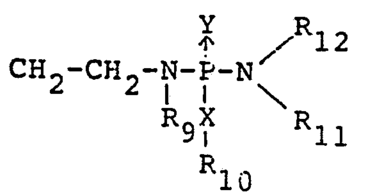 ;

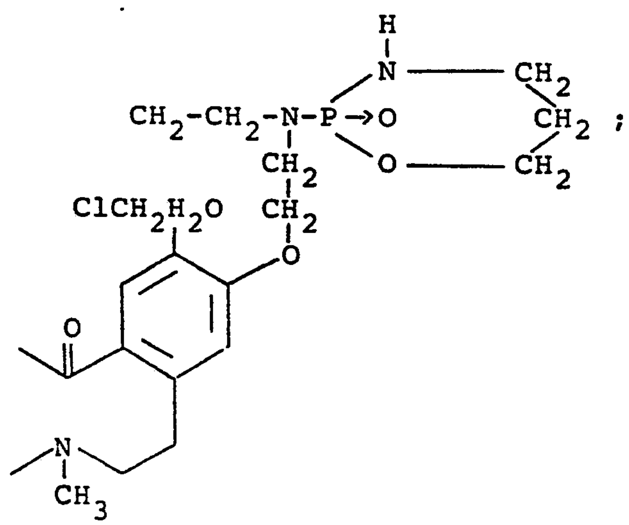 ;

$R_{14}$ = OH; $CH_2Cl$; 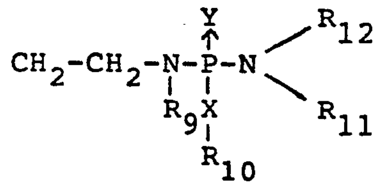 ;

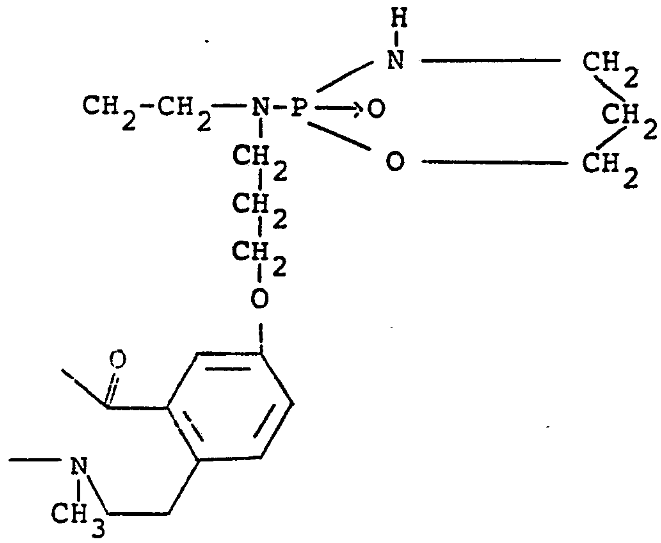 ;

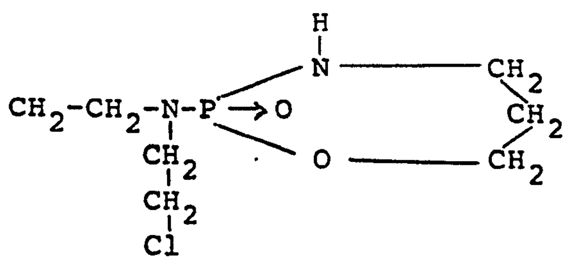 ;

$R_{15}$ = 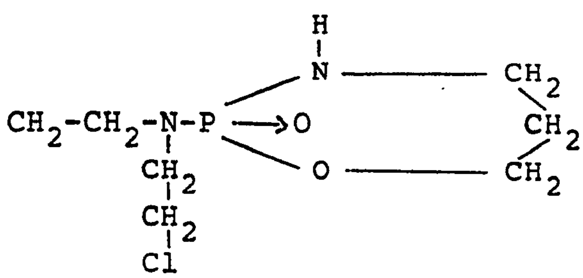 ;

for the production of a drug for strengthening the body's immune system in humans and animals and for the treatment of infections diseases, especially for the treatment of viral, bacterial and mycotic infections.

**Revendication**

Utilisation des substances de la formule générale (I)

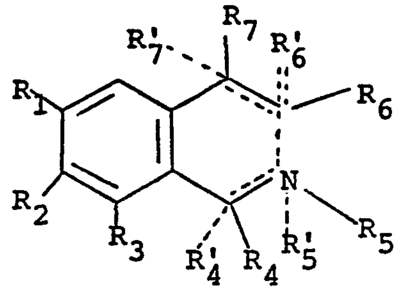

(I)

ou de leurs sels avec acides utilisables en pharmacie, ceux-ci signifiant:

$R_1$ = H; $OCH_3$

$R_2$ = $OCH_3$; OH; 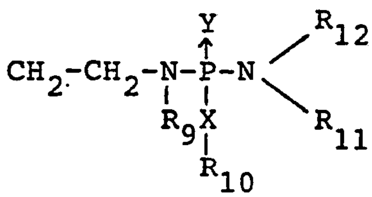 ;

ou $R_1$ et $R_2$ ensemble

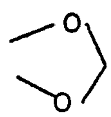

$R_3$ = H; $OCH_3$; 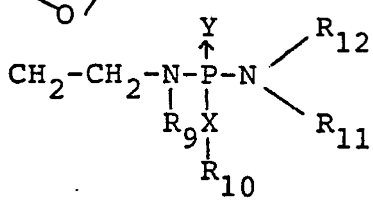

ou $R_2$ et $R_3$ ensemble

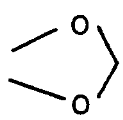

$R_4$ = H;
$R'_4$ = H;
ou $R_4 + T'_4$ = O
ou $R_3$ et $R_4$ ensemble

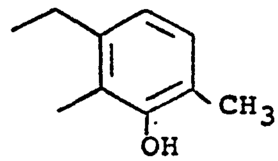

$R_5$ = $CH_3$; OH; 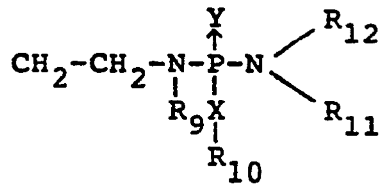 ;

$R'_5$ = $CH_3$ ; OH;
ou $R_4$ et $R_5$ ensemble

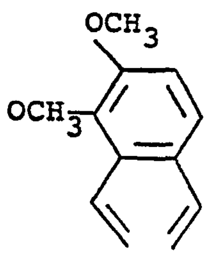 ; 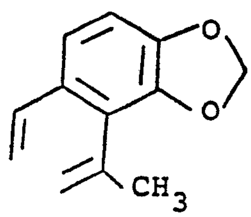 ; 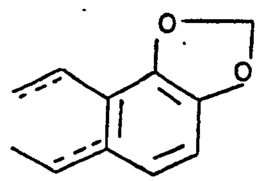 ;

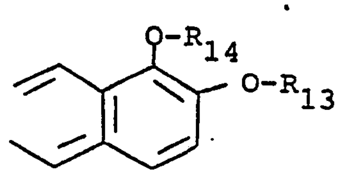 ;

$R_6$ = H;
$R'_6$ = H ou ensemble avec $R_6$ = O
ou $R_5$ et $R_6$ ensemble

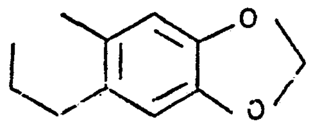 ; 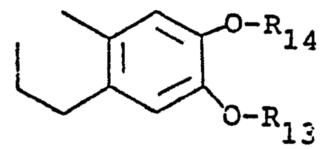

à la réserve qu'au moins un des restes $R_2$, $R_3$ et $R_5$ (seul ou avec $R_4$ ou $R_6$) est un groupe ou contient un groupe,

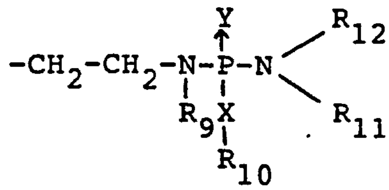

$R_7$ = H;
$R'_7$ = H;
ou $R_6$ et $R_7$ ensemble

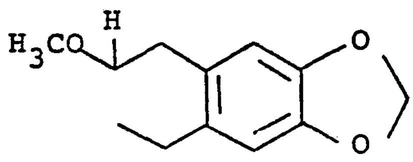 ; 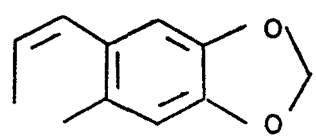 ;

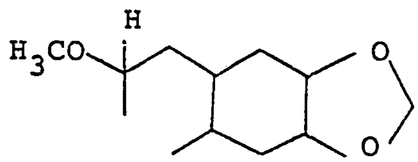 ; 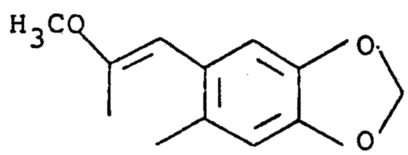 ;

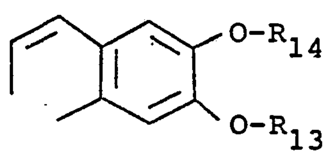 ; 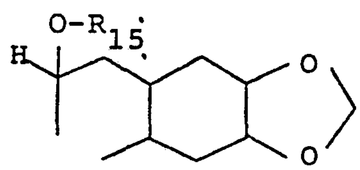 ;

$R_9$ = H; $CH_2$-$CH_2$-Cl; alcaloides:
$R_{10}$ = H; OH; alcaloide;
$R_{11}$ = H; alcaloide;
ou
$R_{11}$ + $R_{12}$ = $CH_2$-$CH_2$;
Y = S; O;
X = NH; O;
$R_{12}$ = H; alcaloide;

$R_{13}$ = OH; 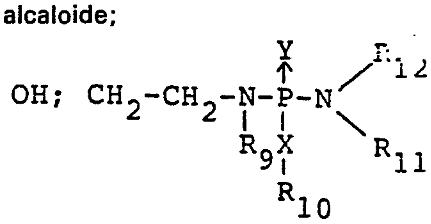 ;

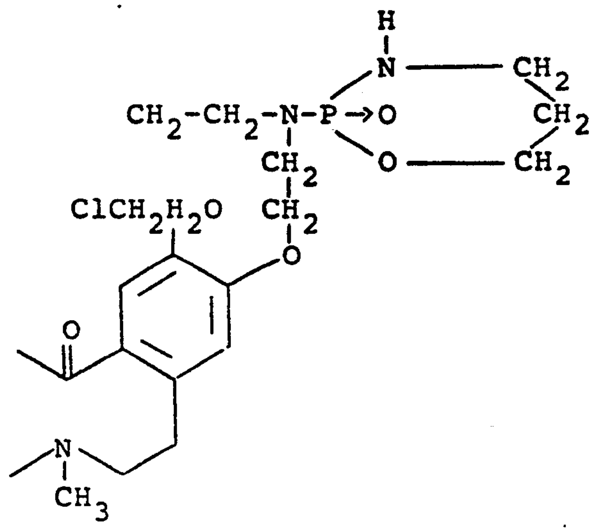

;

$R_{14}$ = OH; $CH_2Cl$; 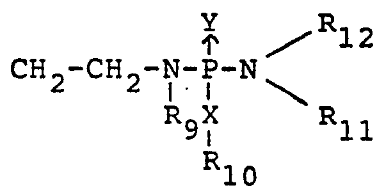 ;

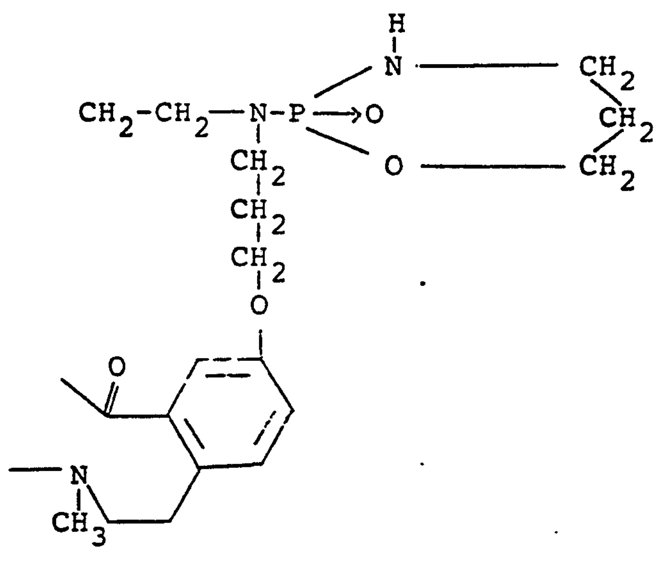

;

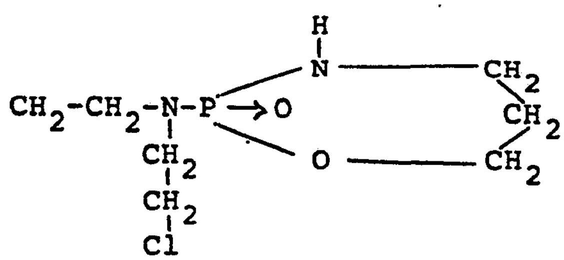 ;

$R_{15}$ = 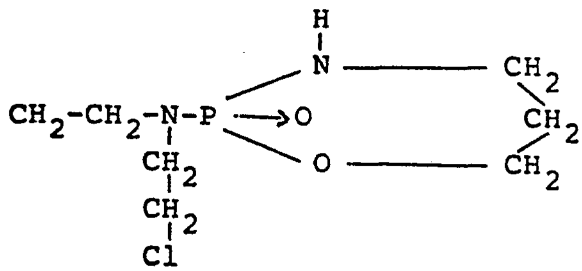 ;

ceci en vue de la fabrication d'un médicament pour fortifier le système de défense du corps humain et animal et pour combattre les maladies infectieuses, en particulier dans le traitement d'affections virales, bactériennes et de mycoses.

Abb. 1. Chelilutin + Thiophosphorsäuretriaziridid

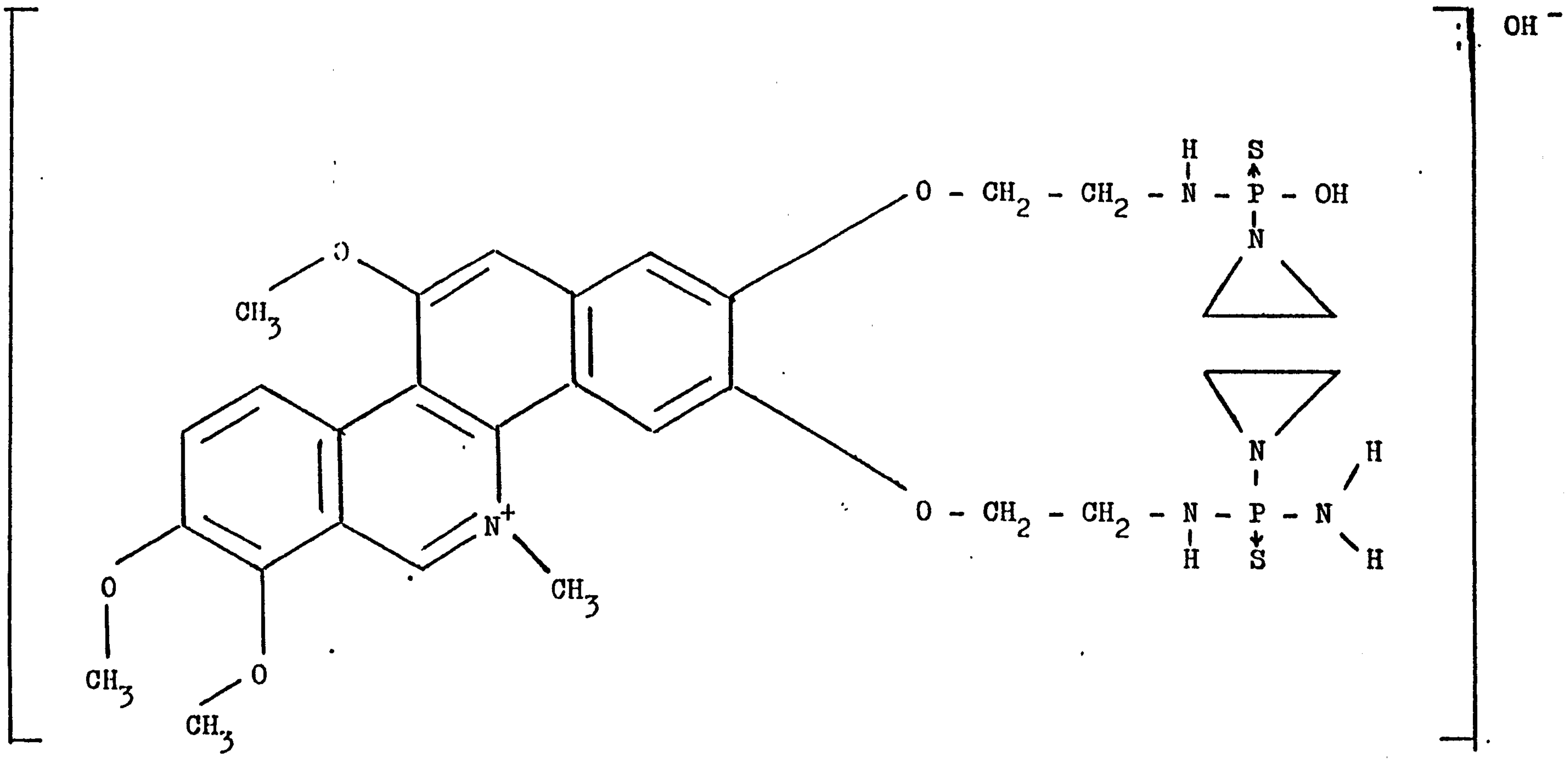

Abb. 2. Chelerythrin + Thiophosphorsäuretriaziridid

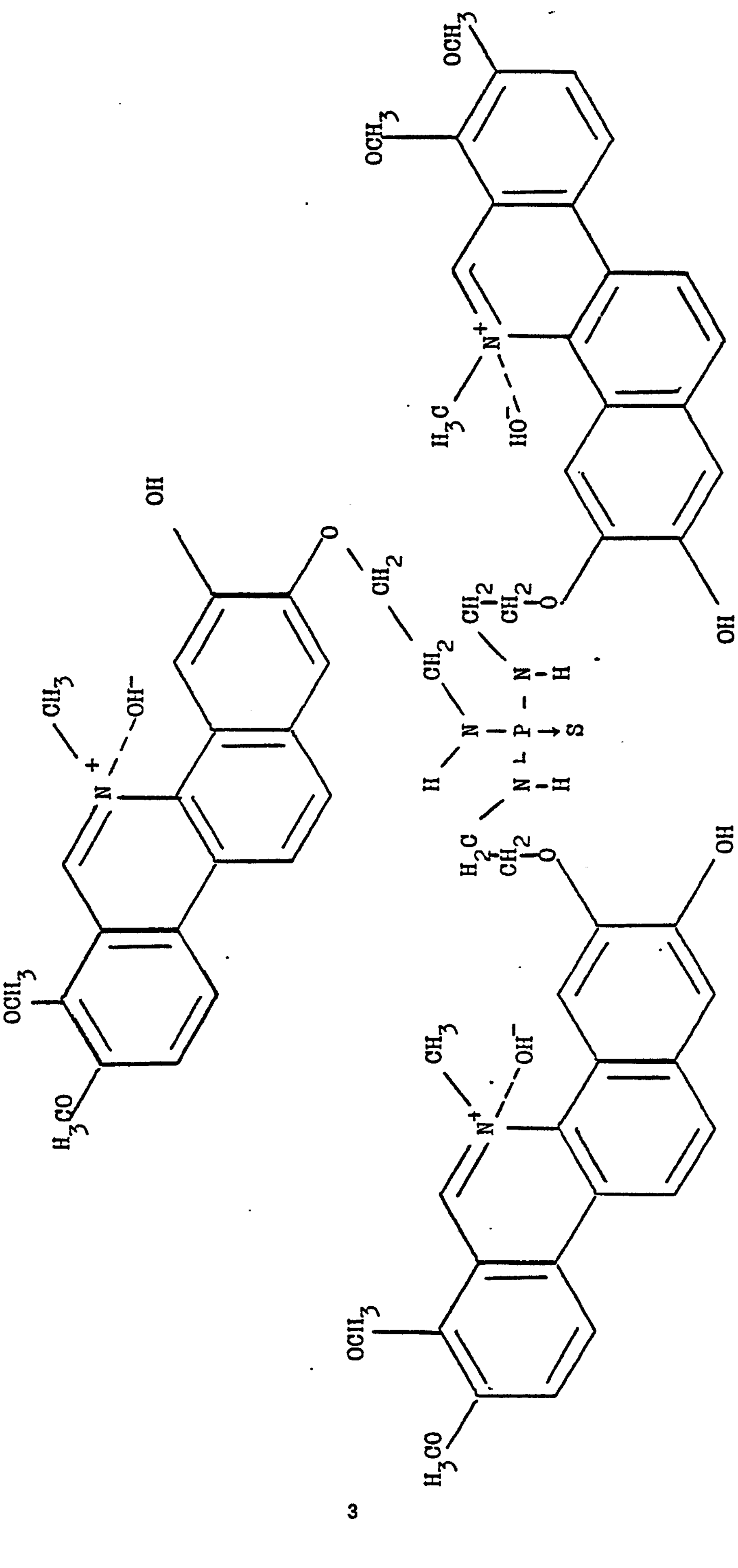

Abb. 3. Coptisin + Thiophosphorsäuretriaziridid

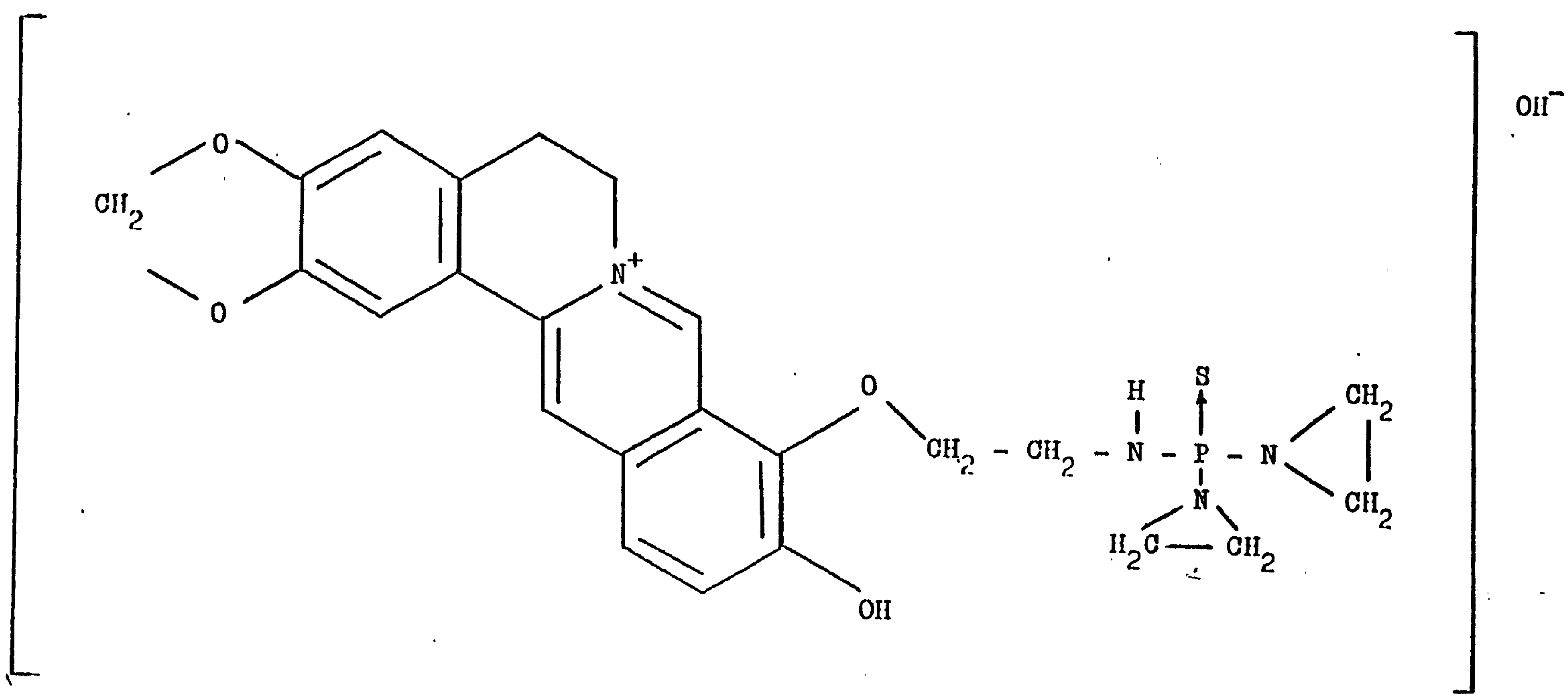

Abb. 4. Chelidonin + Thiophosphorsäuretriaziridid

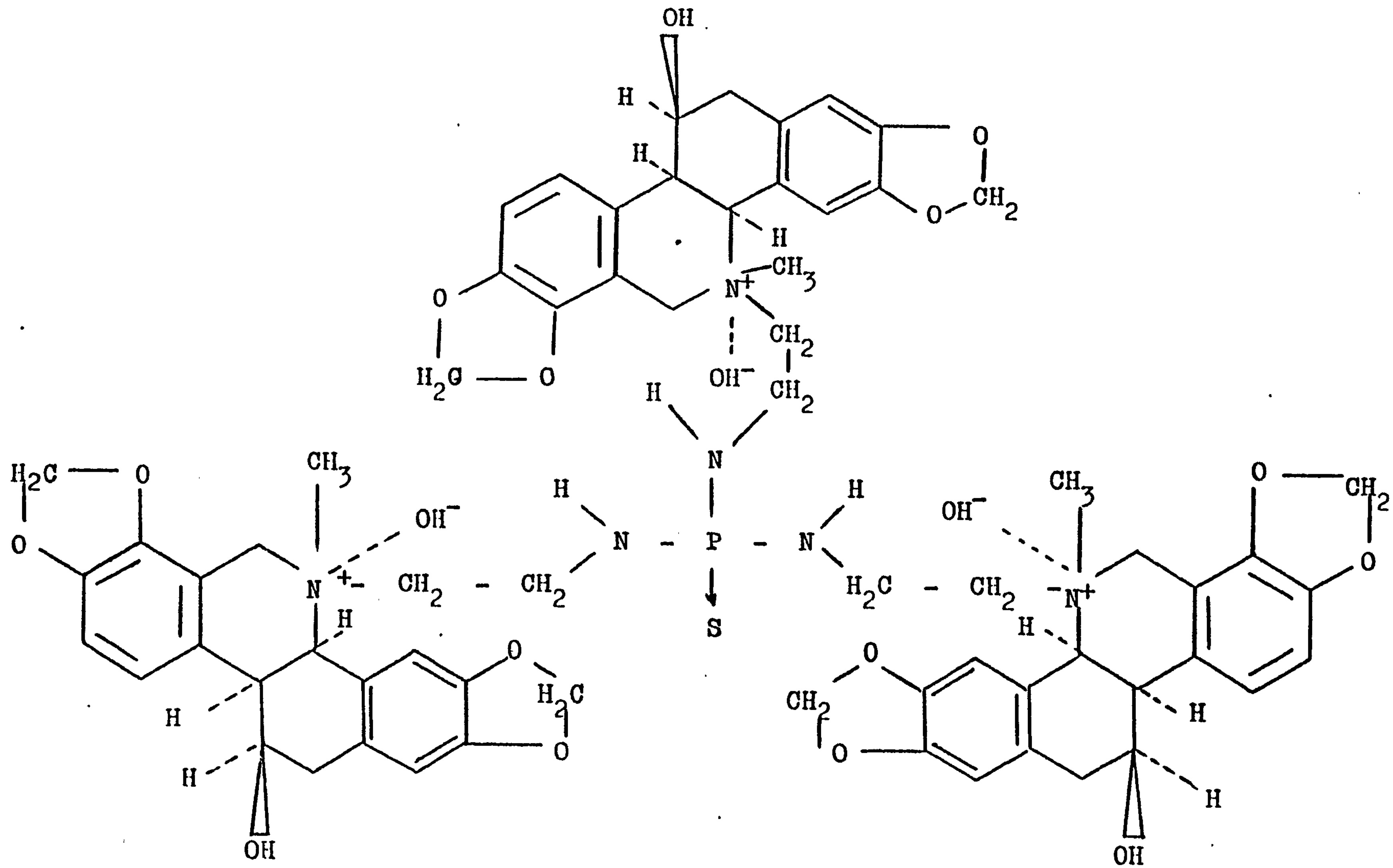

Abb. 5. Protopin + Thiophosphorsäuretriaziridid

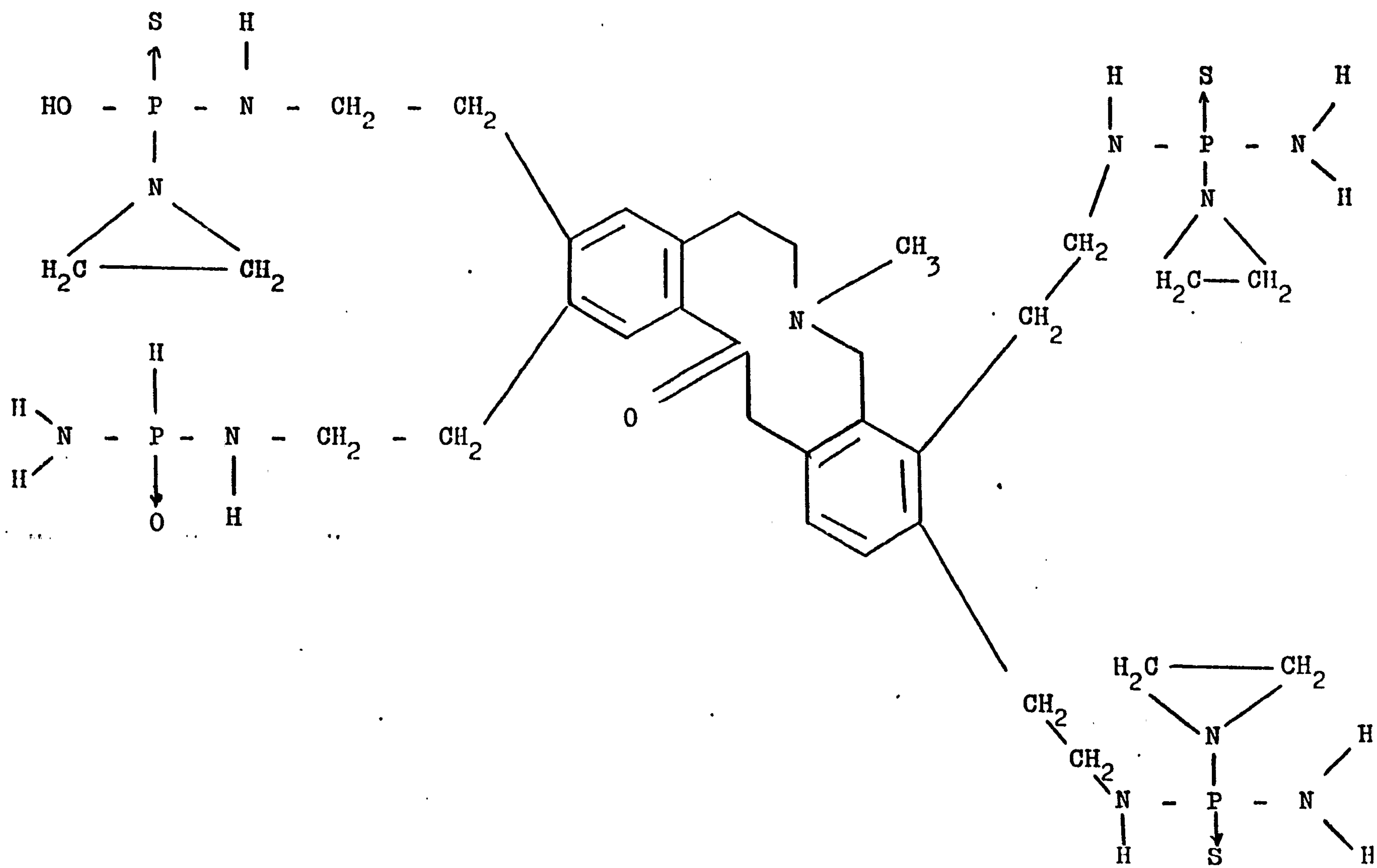

Abb. 6. Reserpine + Thiophosphorsäuretriaziridid

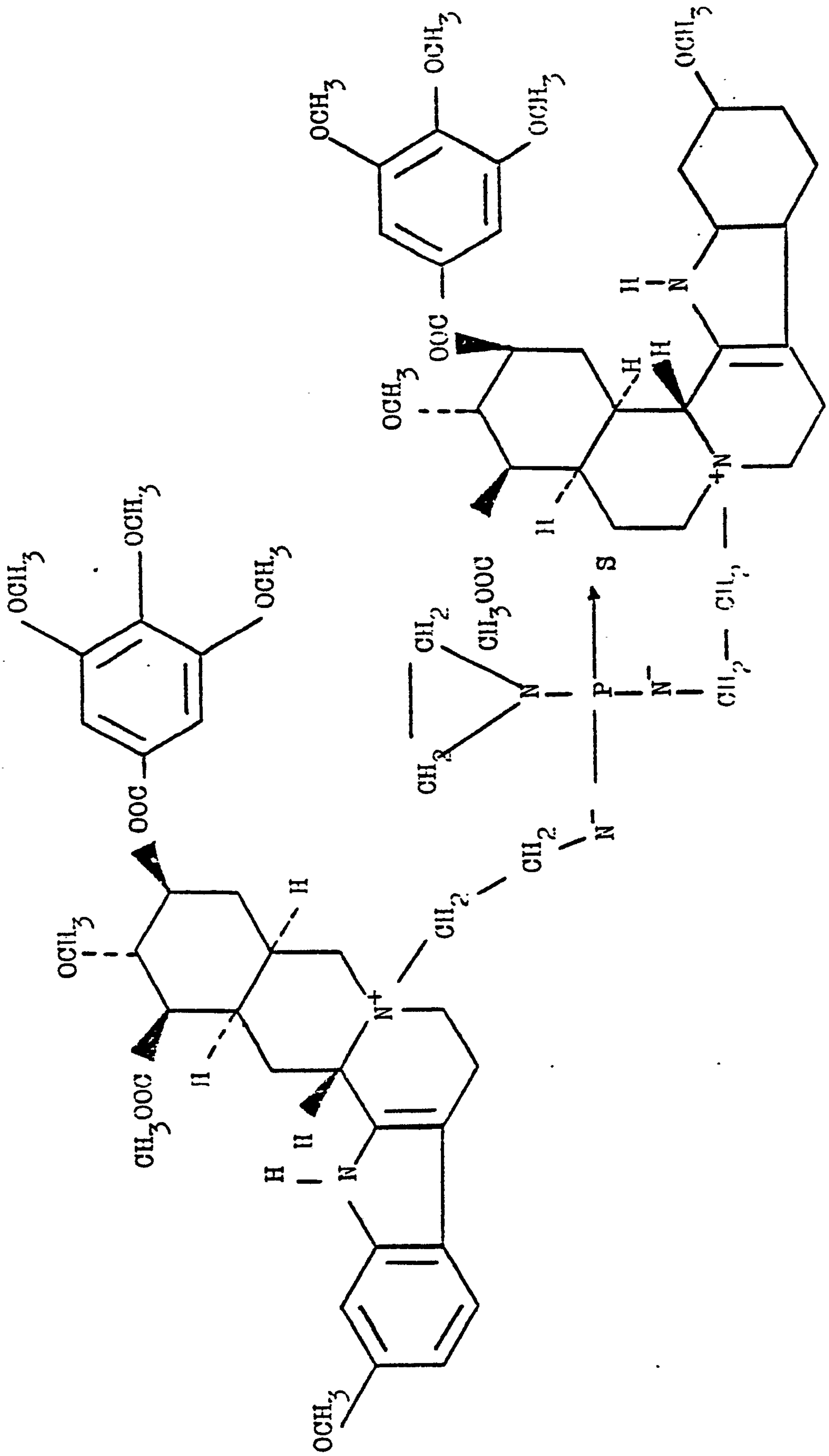

Abb. 7. Reserpine + Thiophosphorsäuretriaziridid

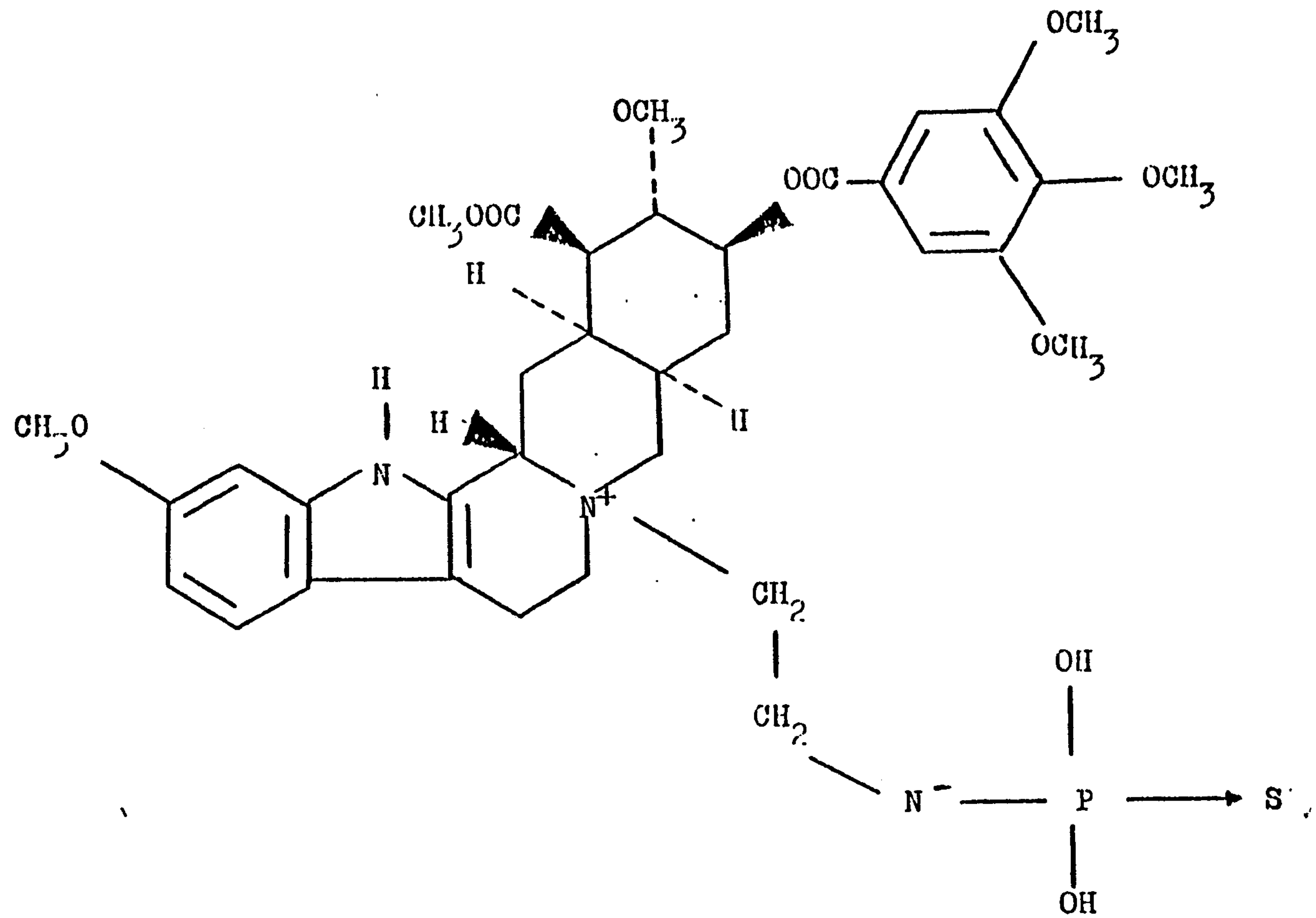

Abb. 8. Coffein + Thiophosphorsäuretriaziridid

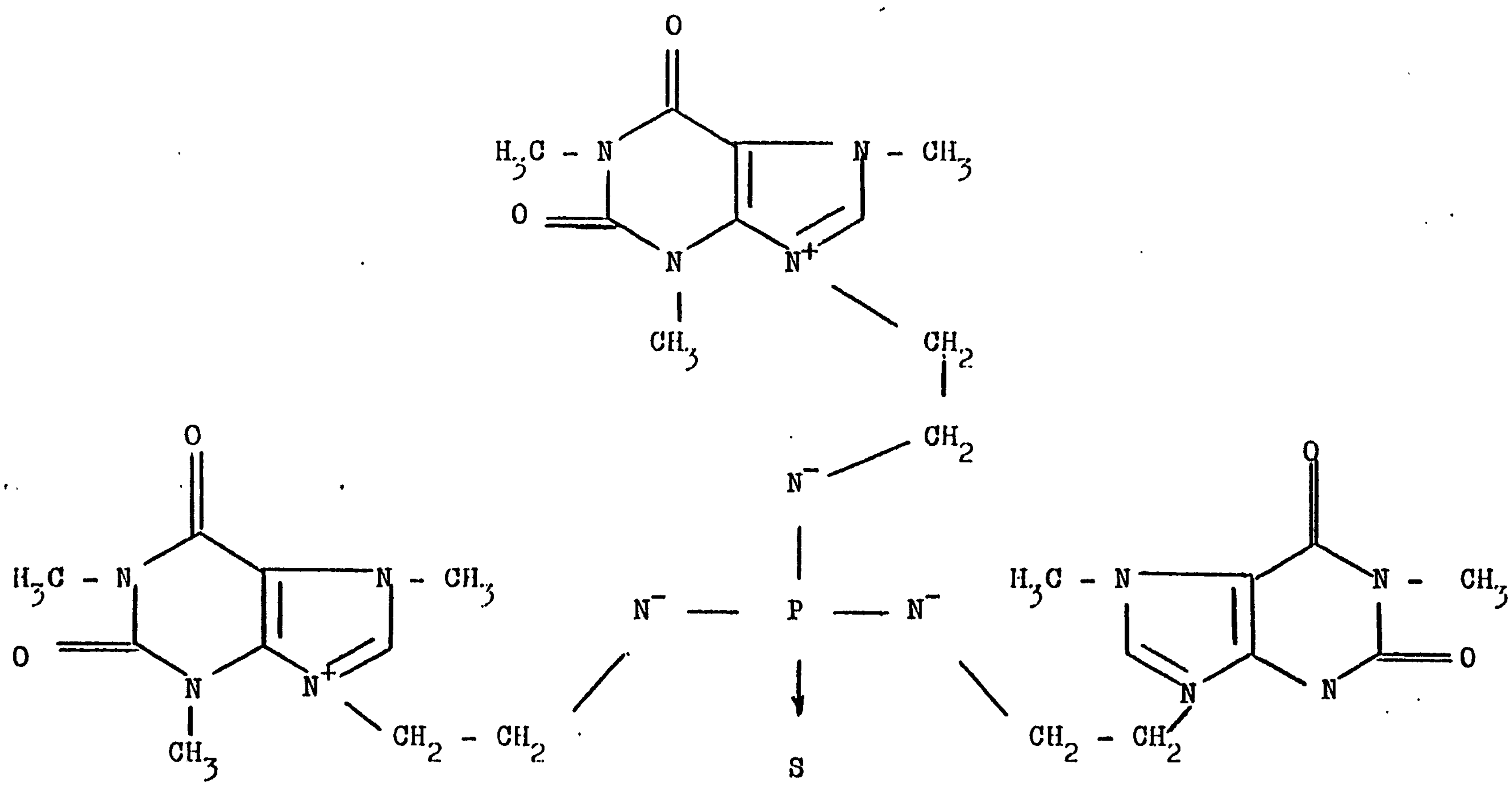

Abb. 9. Narcotine + Thiophosphorsäuretriaziridid

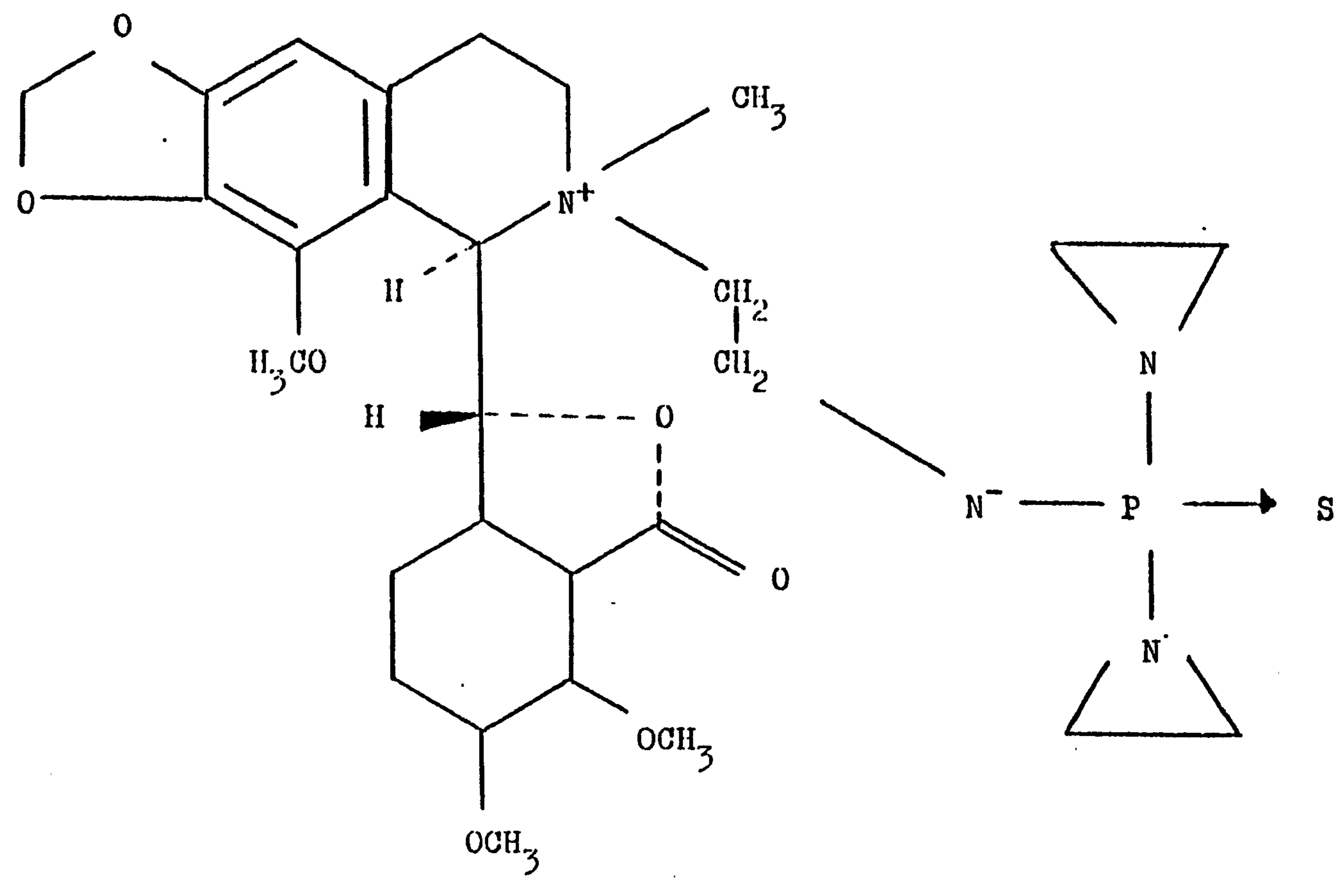

Abb. 10. Aconitine + Thiophosphorsäuretriaziridid

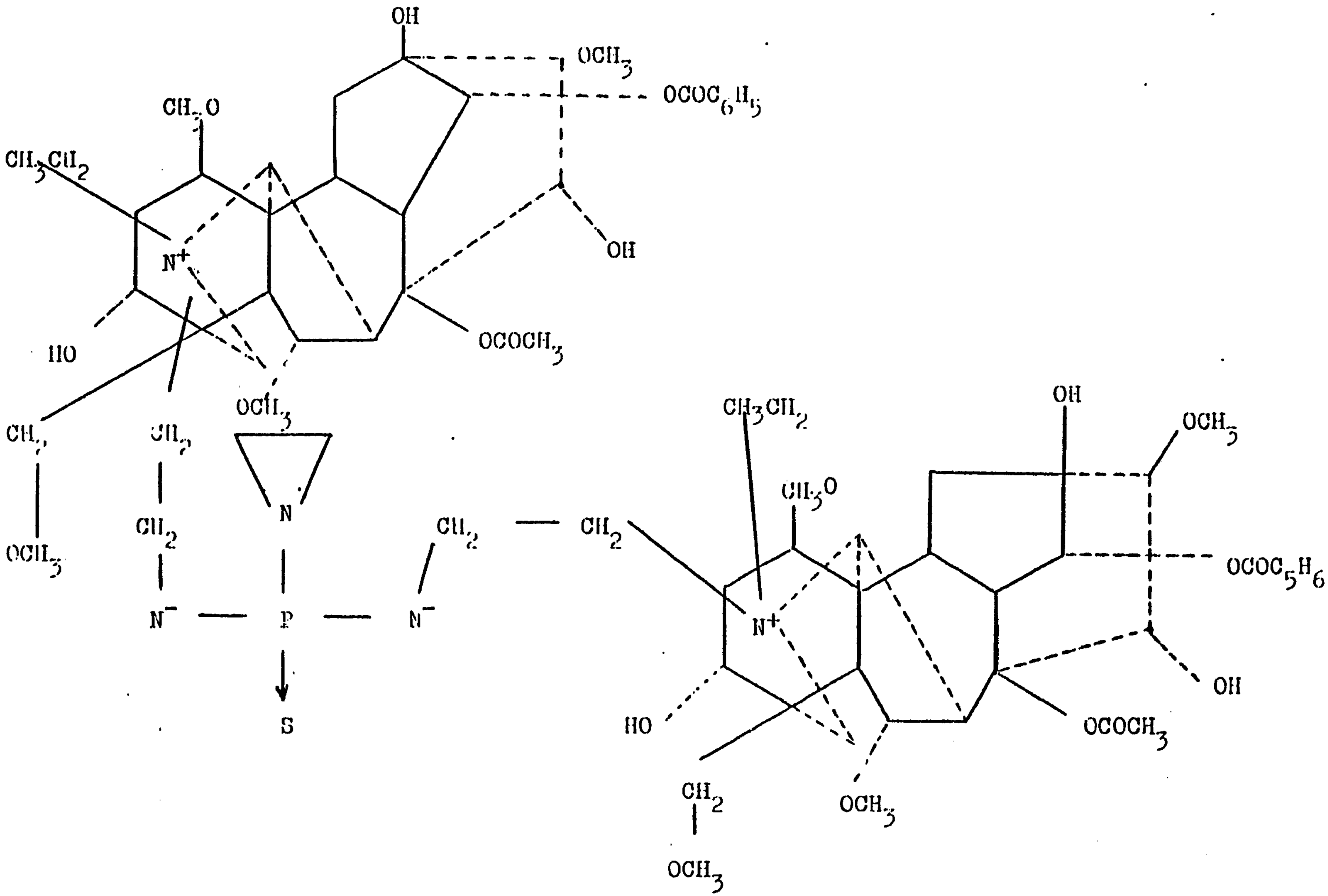

Abb. 11. Aconitine + Thiophosphorsäuretriaziridid

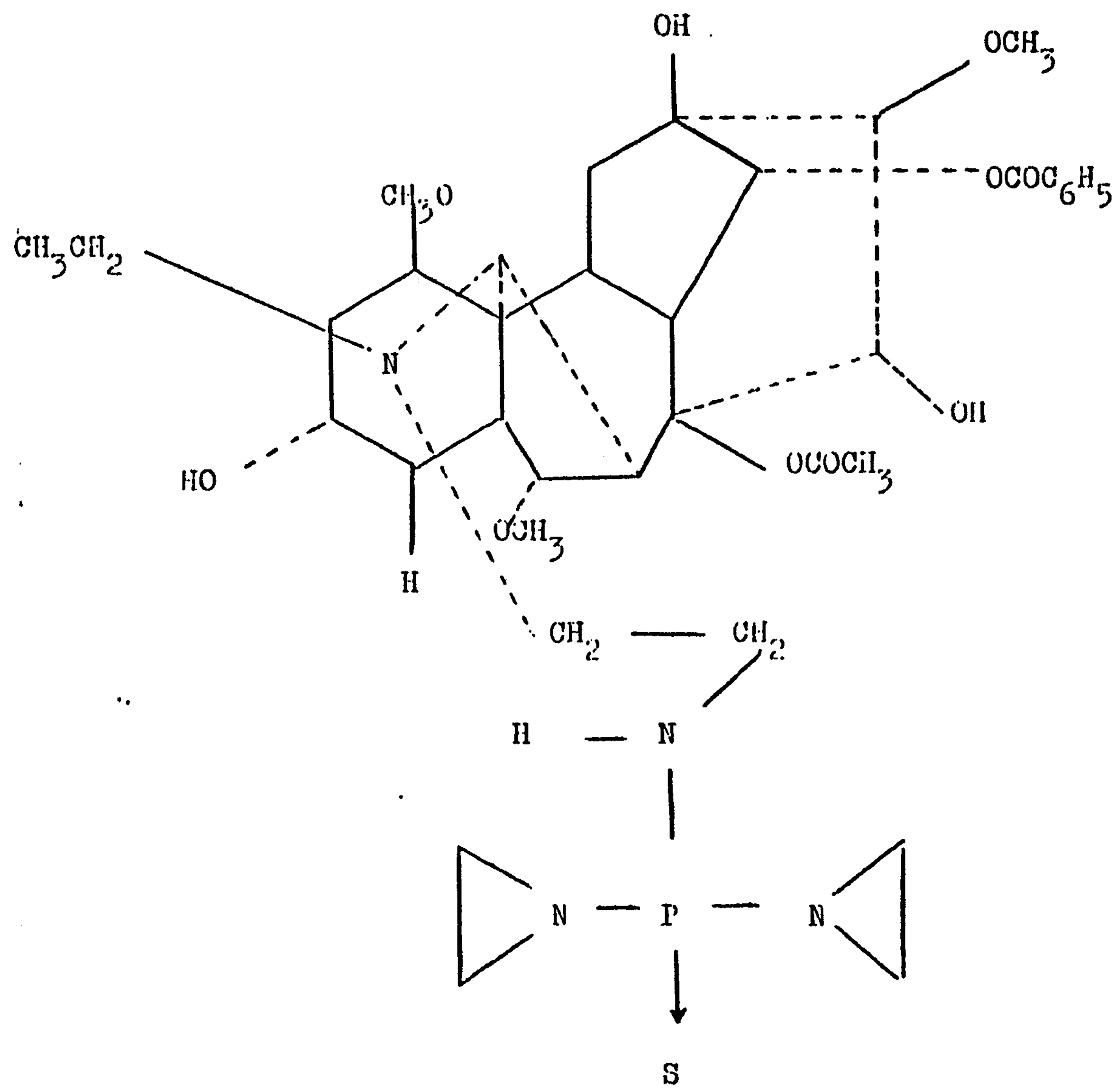

Abb. 12. Aconitine + Thiophosphorsäuretriaziridid

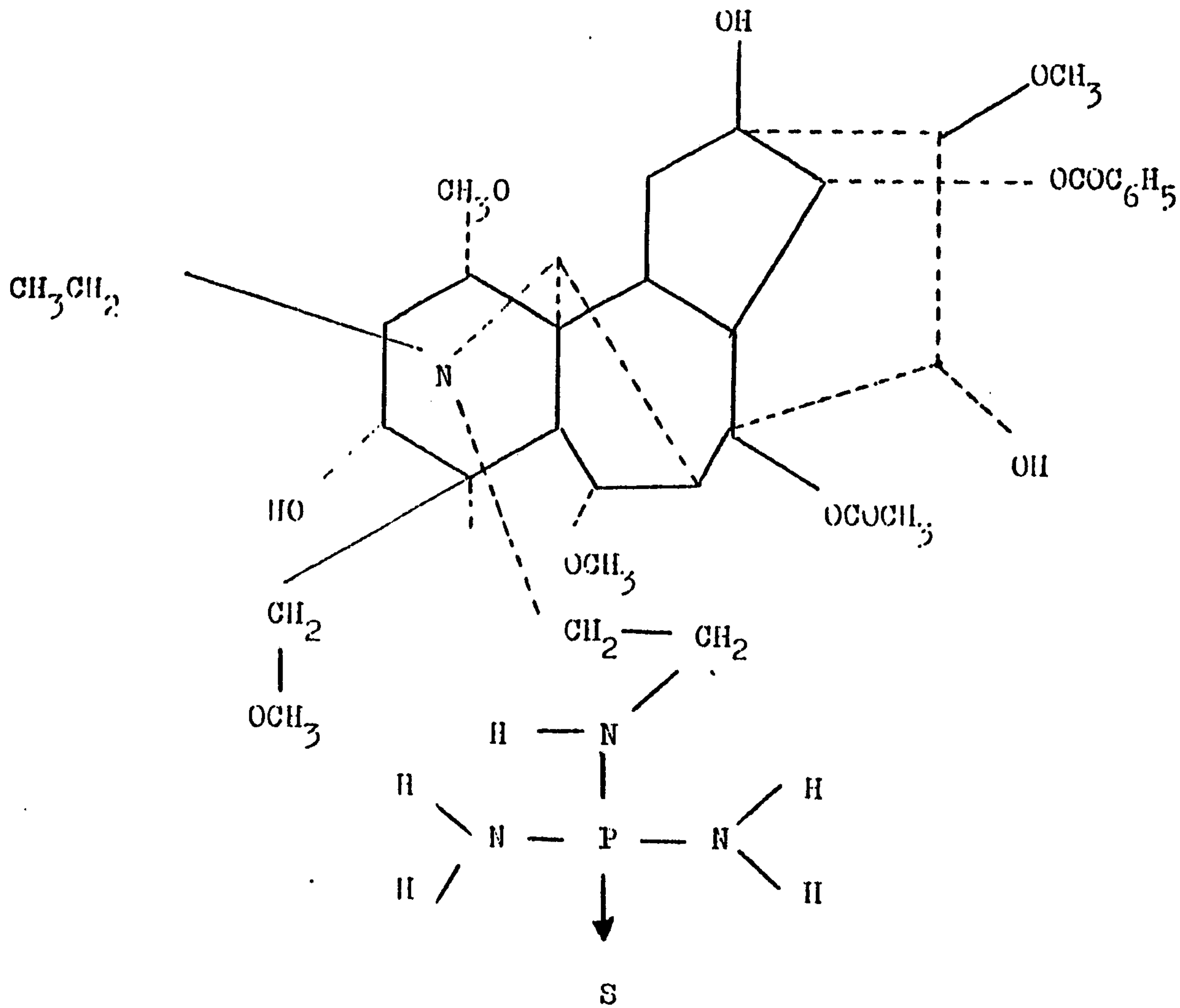

Abb. 13. Pilocarpine + Thiophosphorsäuretriaziridid

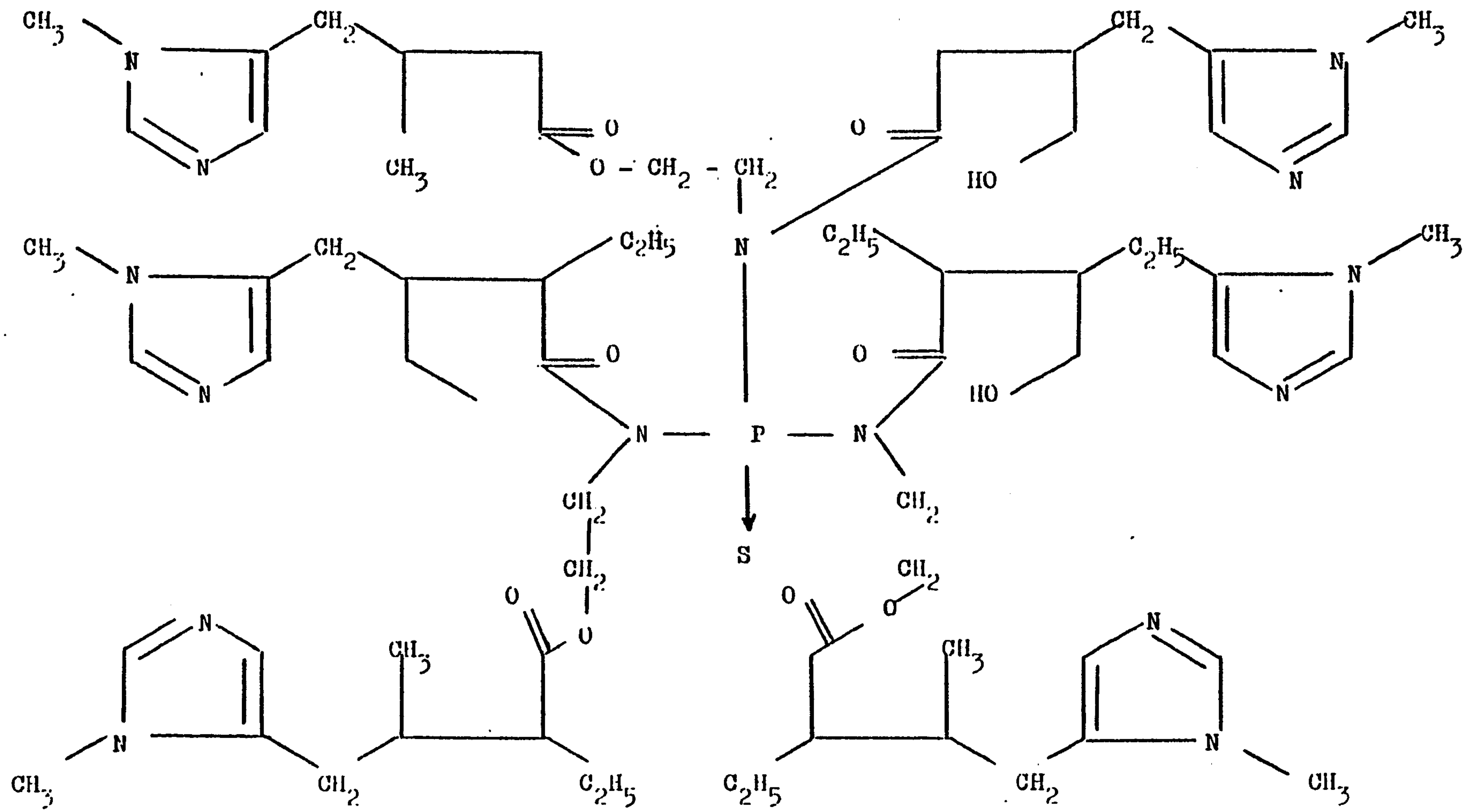

Abb. 14. Allocryptopine + Cyclophosphamide (N, N-Bis- (ß-chloräthyl)-N', O-Propylenphorsäure-esterdiamid)

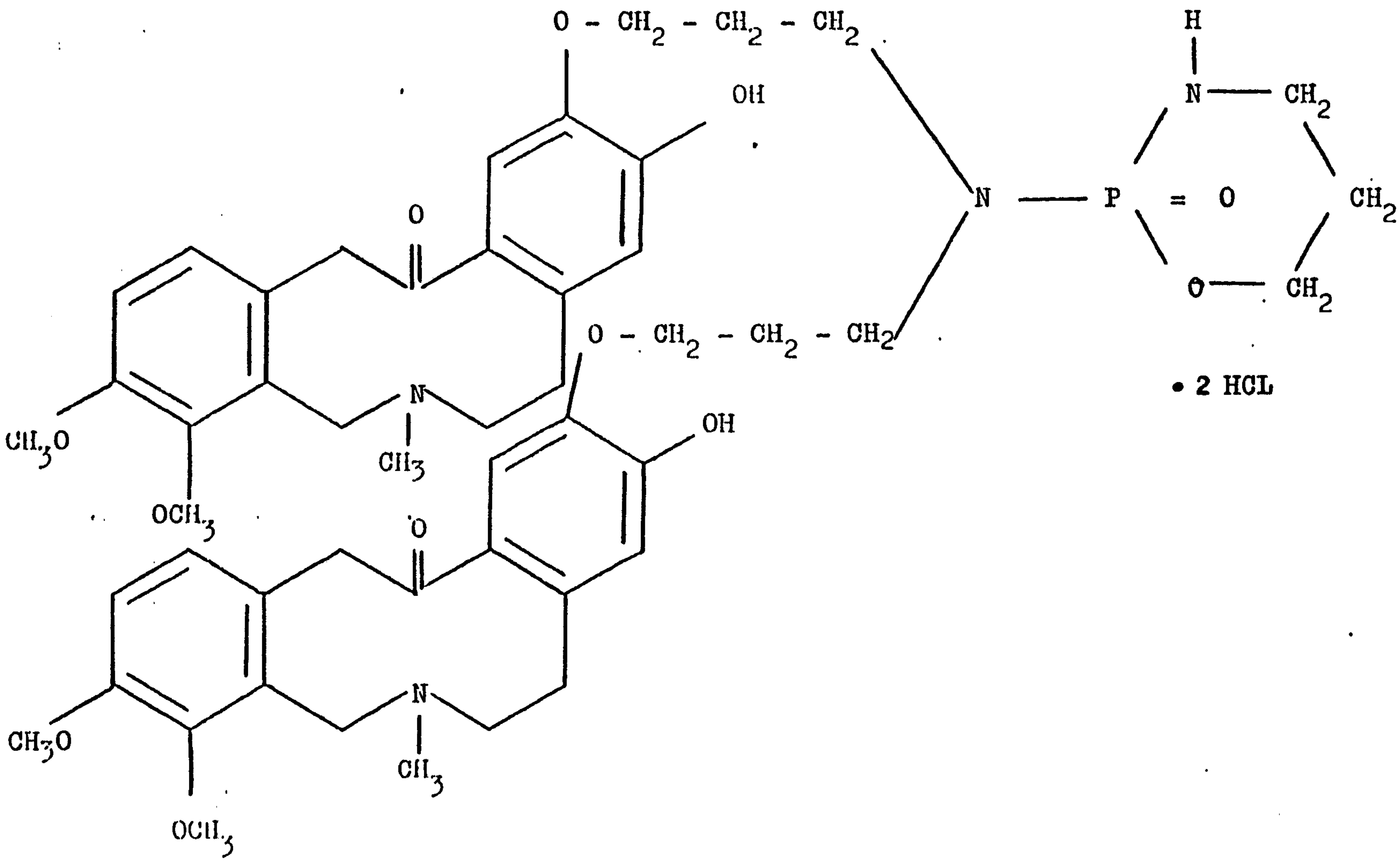

• 2 HCL

Abb. 15. Protopine + Cyclophosphamide (N, N-Bis-(ß-chloräthyl)-N', O-Propylenphosphorsäureesterdiamid)

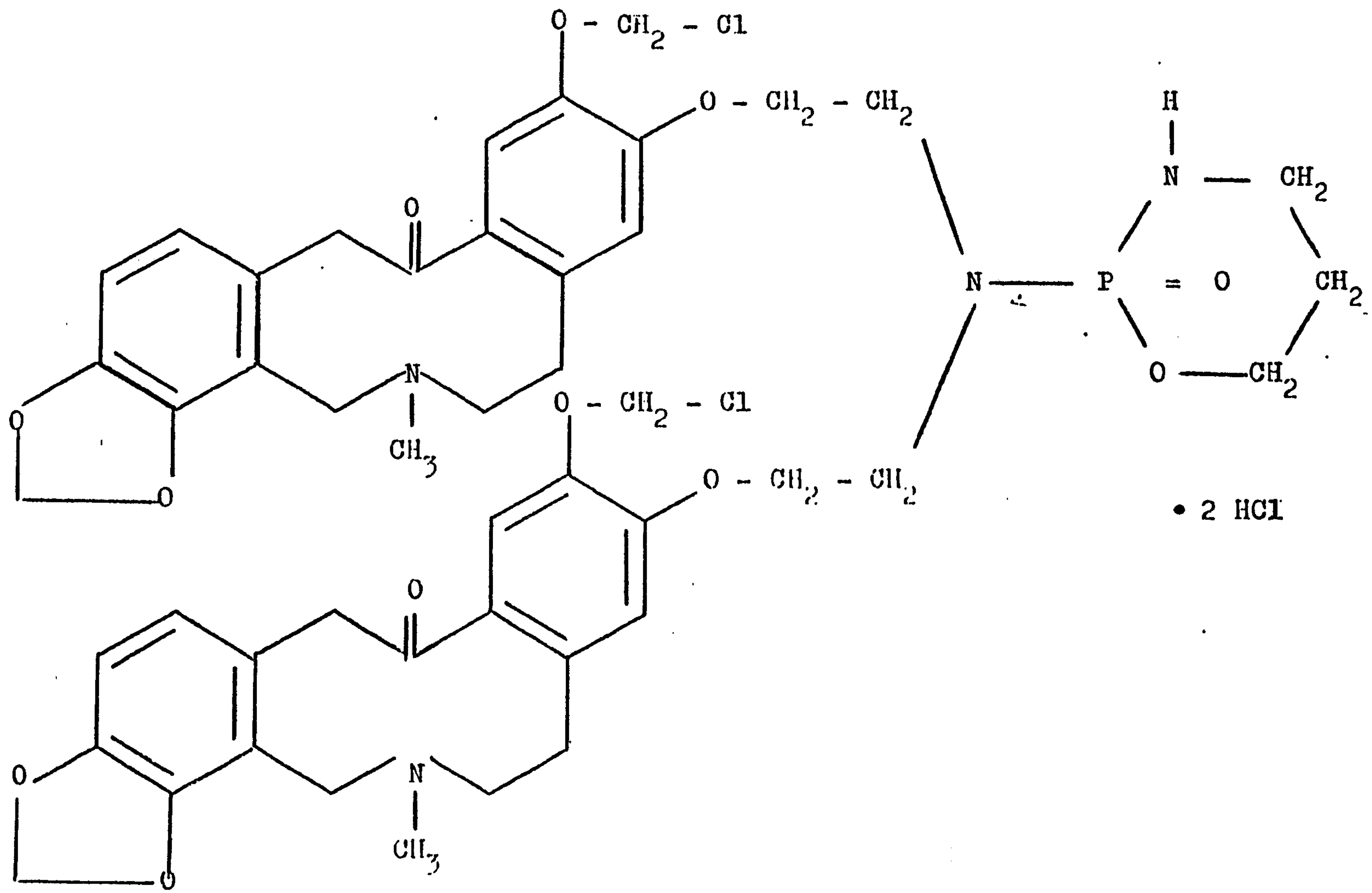

Abb. 15a. Protopine + Cyclophosphamide (N, N-Bis-(ß-chlorathyl)-N', O-Propylenphosphorsäureesterdiamid)

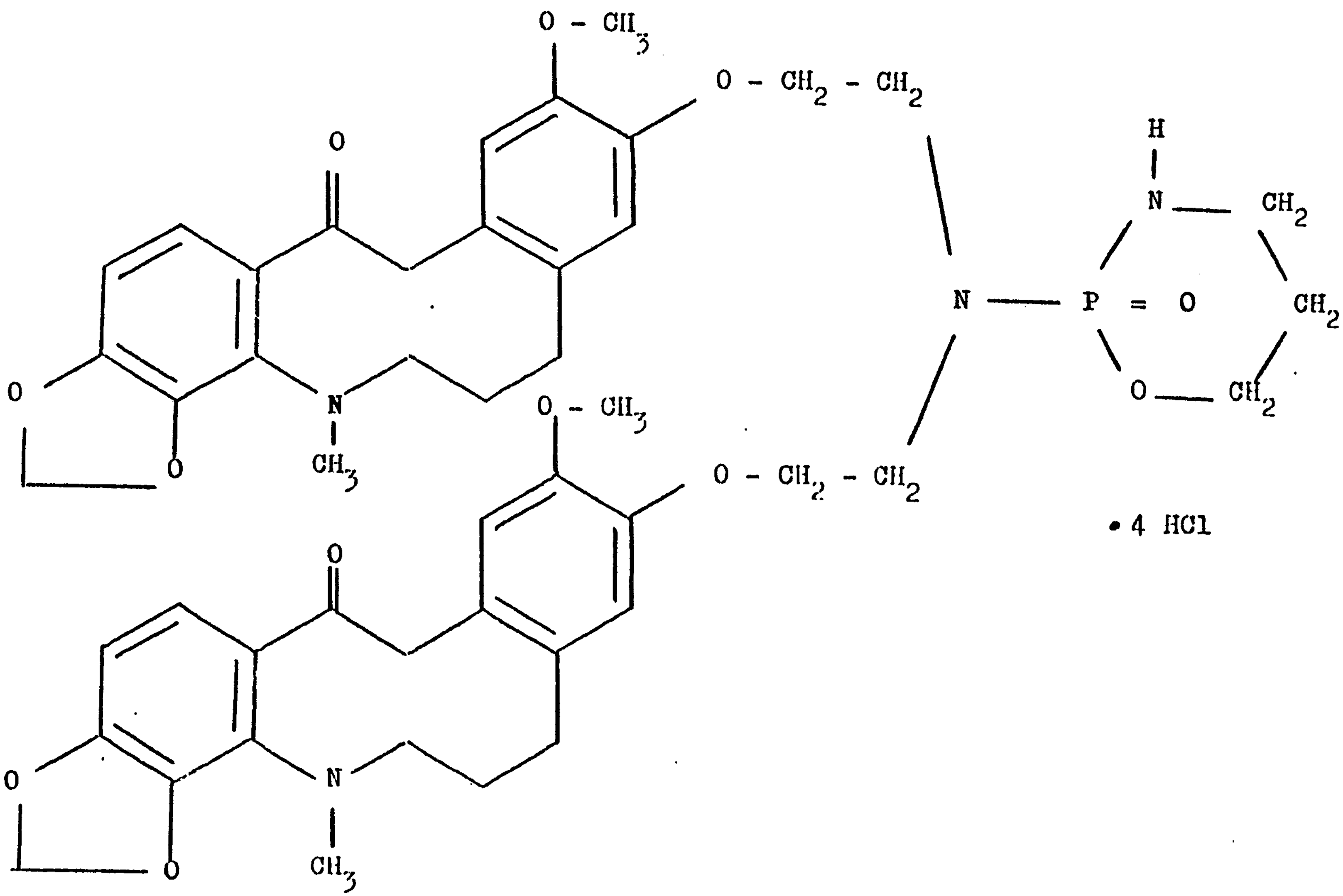

Abb. 15b. Protopine + Cyclophosphamid (N, N-Bis-(ß-chloräthyl)-N', O-Propylenphosphorsäureesterdiamid)

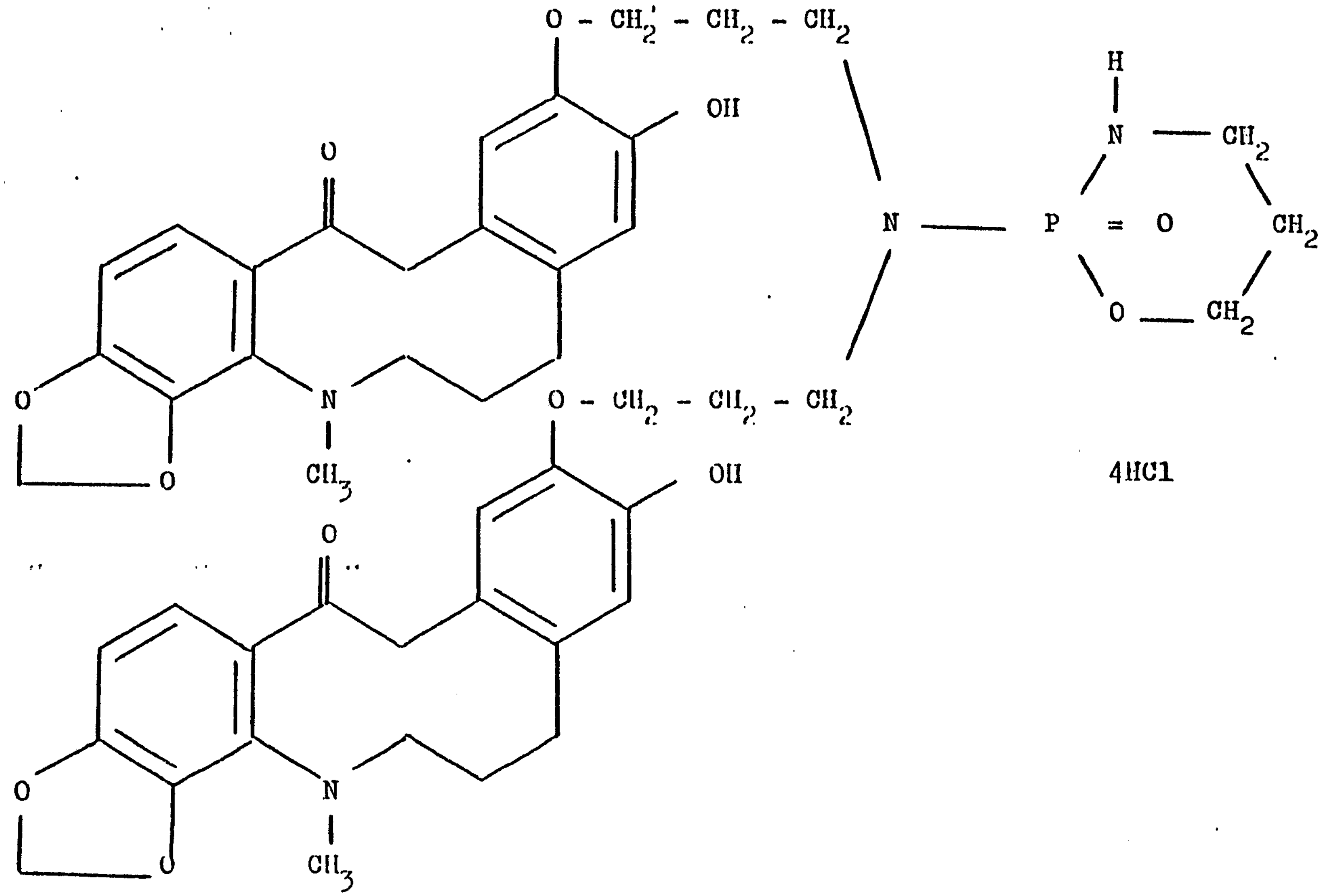

4HCl

Abb. 16. Chelerythrine + Cyclophosphamide (N, N-Bis-(ß-chloräthyl)-N', O-Propylenphosphorsäureester-diamid)

$HCl \cdot 2\ H_2O \cdot$

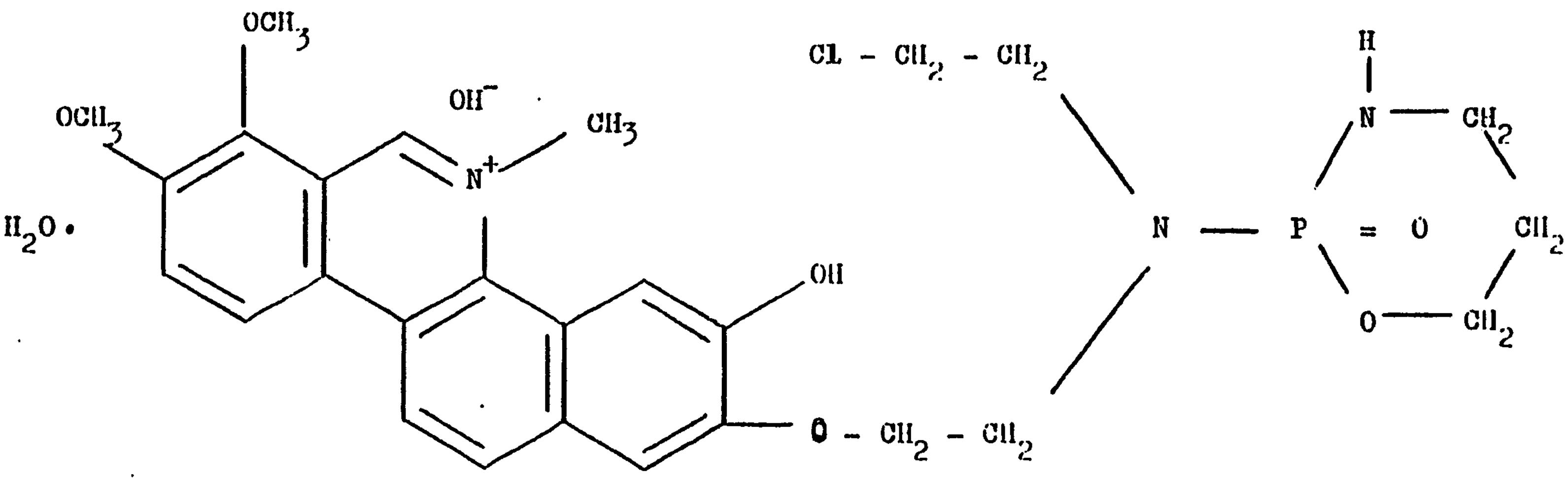

Abb. 17. Chelidonine + Cyclophosphamide (N, N-Bis-(ß-chloräthyl)-N', O-Propylenphosphorsäureesterdiamid)

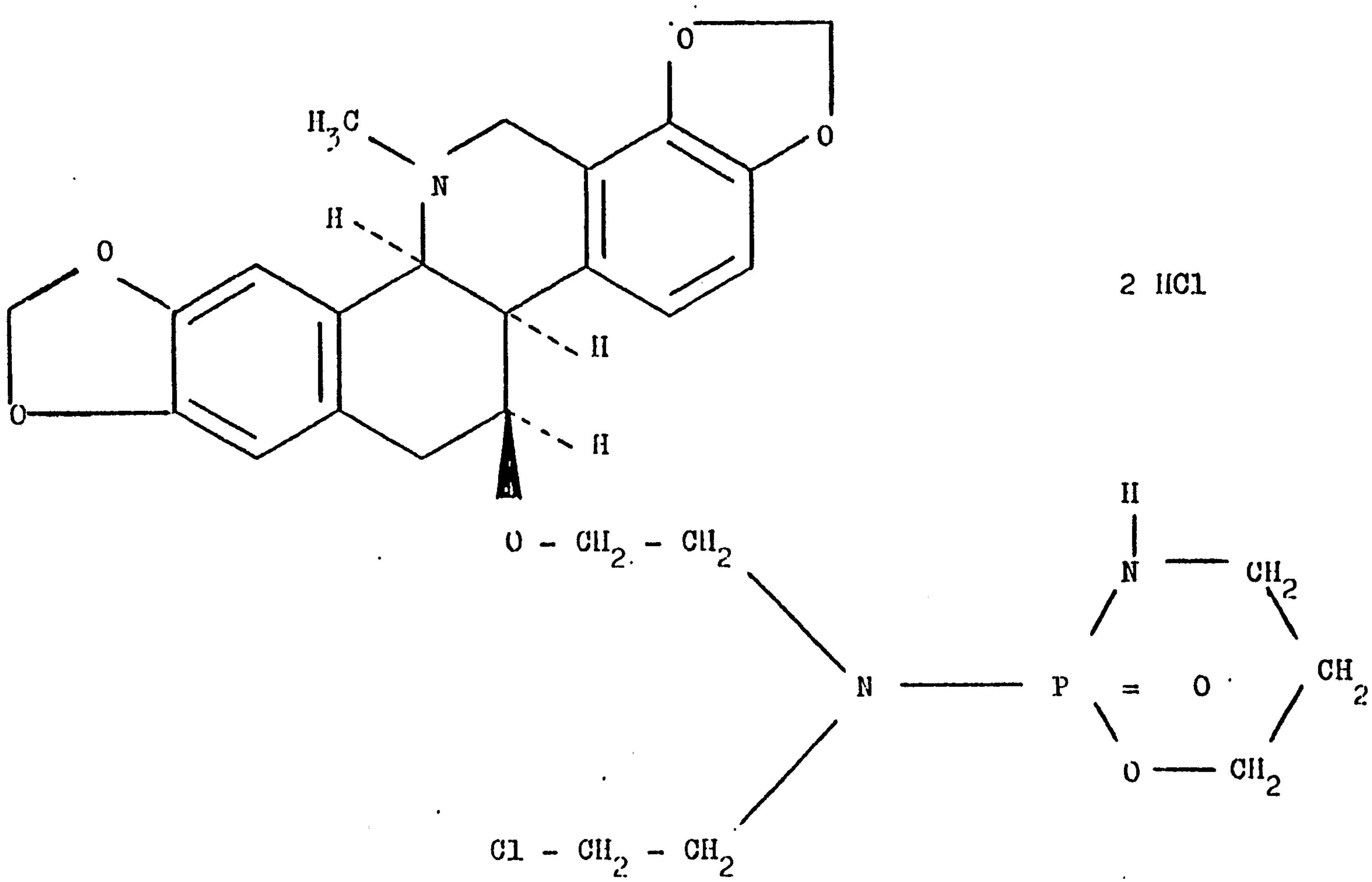

2 HCl